# EUROPEAN PATENT SPECIFICATION

(11) **EP 2 824 185 B1**
(45) Date of publication and mention of the grant of the patent: **10.04.2019**
(21) Application number: 14177029.7
(22) Date of filing: 19.02.2004
(51) Int. Cl.: A61P 27/00

(54) **MATERIALS AND METHODS FOR TREATING DISORDERS OF THE EAR**
MATERIALIEN UND VERFAHREN ZUR BEHANDLUNG VON KRANKHEITEN DES OHRS
MATERIAUX ET PROCEDES DE TRAITEMENT DES TROUBLES DE L'AUDITION

(30) Priority: 24.02.2003 US 373249
(43) Date of publication of application: 14.01.2015
(62) Divisional of application: 04712832.7
(73) Proprietor: GenVec, Inc., Gaithersburg, MD 20878 (US)
(72) Inventor: BROUGH, Douglas E., Gaithersburg, MD 20879 (US)
(74) Representative: Hoeger, Stellrecht & Partner Patentanwälte mbB

(56) References cited:
- WO-A-00/73764
- US-A- 5 851 806
- LUEBKE A E ET AL: "Cochlear function and transgene expression in the guinea pig cochlea, using adenovirus- and adeno-associated virus-directed gene transfer.", HUMAN GENE THERAPY 1 MAY 2001, vol. 12, no. 7, 1 May 2001 (2001-05-01), pages 773-781, XP002732915, ISSN: 1043-0342
- ZHENG J LISA ET AL: "Overexpression of Math1 induces robust production of extra hair cells in postnatal rat inner ears", NATURE NEUROSCIENCE, vol. 3, no. 6, June 2000 (2000-06), pages 580-586, XP002291282, ISSN: 1097-6256
- ZUO JIAN: "Transgenic and gene targeting studies of hair cell function in mouse inner ear", JOURNAL OF NEUROBIOLOGY, vol. 53, no. 2, November 2002 (2002-11), pages 286-305, XP002291284, ISSN: 0022-3034
- KAWAMOTO KOHEI ET AL: "Math1 gene transfer generates new cochlear hair cells in mature guinea pigs in vivo.", JOURNAL OF NEUROSCIENCE, vol. 23, no. 11, 1 June 2003 (2003-06-01), pages 4395-4400, XP002291285, ISSN: 0270-6474
- SHOU JIANYONG ET AL: "Robust generation of new hair cells in the mature mammalian inner ear by adenoviral expression of Hath1.", MOLECULAR AND CELLULAR NEUROSCIENCE, vol. 23, no. 2, June 2003 (2003-06), pages 169-179, XP002291286, ISSN: 1044-7431
- ZINE AZEL: "Molecular mechanisms that regulate auditory hair-cell differentiation in the mammalian cochlea.", MOLECULAR NEUROBIOLOGY, vol. 27, no. 2, April 2003 (2003-04), pages 223-237, XP009034956, ISSN: 0893-7648

## Description

### FIELD OF THE INVENTION

The invention relates to a pharmaceutical composition for altering the sensory perception of an animal.

### BACKGROUND OF THE INVENTION

The ear is a complex organ comprising a labyrinth of structures responsible for hearing and balance. Perception of both hearing and balance lies in the ability of inner ear structures to transform mechanical stimuli to impulses recognized by the brain. The sensory receptors responsible for hearing are located in the cochlea, a spiral-shaped canal filled with fluid. Within the cochlea is the organ of Corti, which is lined with columnar sensory hair cells bridging the basilar membrane and the tectorial membrane. As sound waves pass through the organ of Corti, the basilar membrane vibrates causing the hair cells to bend back and forth. The movement depolarizes the hair cell, leading to release of neurotransmitters to the auditory nerve, which carries the impulse to the brain.

Balance and orientation is mediated by the vestibular system of the inner ear. The vestibular system comprises the utricle and saccule, which detect linear motion, and the semi-circular canals, which detect circular motion. In each region of the vestibular system, movement of the head region creates disruption of fluid or small calcium stones in the vestibular organs resulting in movement of hair cells. Nerve impulses created from the bending of hair cells are transmitted to the brain, thereby providing information as to the body's position.

In both hearing and balance, mechanical stimuli are translated into neural signals by sensory hair cells, damage to which is responsible for many types of hearing loss and balance disorders. Mechanical damage by, for example, loud noises, bend cochlear hair cells to the point that the hair cell can no longer transduce signals to the auditory nerves. As mammalian hair cells do not regenerate naturally, permanent hearing loss can occur if hair cells are damaged. Aside from acoustic trauma, which is the predominant cause of hearing impairment, hearing loss also is attributed to hereditary syndromes, bacterial or viral infections, use of prescription drugs, and presbycusis (hearing loss associated with old age). Likewise, balance disorders, especially vestibular disorders, have been caused by infection, head injury, pharmaceutical use, and age.

Hearing loss and balance disorders are indiscriminate among the world's population, with a majority of the population expected to experience some reduction in hearing capacity in their lifetime. In fact, more than 28 million Americans are deaf or hearing impaired (ranging from minor loss of sensitivity to complete loss of hearing), with 80% experiencing irreversible hearing loss. Indeed, approximately 54% of the population over the age of 65 years lives with impaired hearing (Better Hearing Institute, 1999, reported by the American Speech-Language-Hearing Association). Options for treating hearing loss are few. Most common treatments involve hearing aids and cochlear implants. Treatment options for balance disorders include balance retraining and physical therapy. However, such therapies will likely be required over extended periods of time if the disorder is progressive. Presently, there is no proven, effective drug treatment for disorders involving loss or damage of sensory hair cells in the ear.

Given the prevalence of hearing and balance disorders and the lack of efficient treatment options, there remains a need for an effective method of modulating inner ear-mediated sensory perception of an animal, which would serve as a prophylactic and therapeutic treatment of disorders associated with the ear, in particular those disorders associated with destruction or loss of sensory hair cells, such as hearing loss and balance disorders. Accordingly, the invention provides materials and methods for changing the sensory perception of an animal. This and other advantages of the invention will become apparent from the detailed description provided herein.

WO 00/73764 A2 discloses compositions and methods for the therapeutic use of an atonal-associated nucleic acid or aminoacid sequence. The document also discloses an animal having a heterologous nucleic acid sequence replacing an allele of an atonal-associated nucleic acid sequence under conditions wherein said heterologous sequence inactivates said allele.

### BRIEF SUMMARY OF THE INVENTION

The present invention relates to a pharmaceutical composition according to claim 1. Preferred embodiments of the invention are indicated in the dependent claims.

### DETAILED DESCRIPTION OF THE INVENTION

Sensory perception requiring transformation of mechanical stimuli to nerve impulses, such as perception of sound or position (balance), are dependent on the efficient functioning of sensory hair cells. In most mammals, hair cells are fully differentiated upon birth and do not regenerate. Thus, damage to hair cells throughout the lifespan of a mammal is irreversible (Hawkins, Adv. Oto-Rhino-Laryngol., 20, 125-141 (1973)). It was previously impossible to correct a loss of sensory hair cells in the ear. The invention is predicated, at least in part, on the surprising discovery that sensory hair cells can be generated by delivering to the sensory epithelium a nucleic acid sequence encoding a transcription factor of the atonal-associated family of proteins. An atonal-associated factor promotes the differentiation of non-sensory cells of the sensory epithelium, i.e., supporting cells, into sensory hair cells. The ability to transform a non-sensory cell of the inner ear into a functional sensory hair cell represents a major breakthrough in improving perception of environmental stimuli. Surprisingly, atonal-associated factors convert supporting cells into hair cells in mature, differentiated sensory epithelium. In other words, the inventive composition allows for the generation of a hair cell from a differentiated progenitor cell, implying that stem cells are not required to replenish a population of hair sensory cells. The generation of sensory hair cells in the inner ear is exploited to modulate the sensory perception of an animal. Ideally, the inventive composition prophylactically or therapeutically treats an animal, preferably a mammal (e.g., a human), for at least one disorder associated with loss or damage of sensory hair cells, e.g., disorders of the ear associated with damage of sensory hair cells (such as hearing loss or balance disorders). The inventive composition also is useful in maintaining a level of sensory perception, i.e., controlling the loss of perception of environmental stimuli caused by, for instance, the aging process.

### Sensory Perception

In particular, the invention provides a pharmaceutical composition changing the sensory perception of an animal. The composition is to be administered to the inner ear an expression vector (e.g., expression viral vector) comprising a nucleic acid sequence encoding an atonal-associated factor. The nucleic acid sequence is expressed to produce the atonal-associated factor, which results in generation of sensory hair cells that allow perception (or recognition) of stimuli in the inner ear. By "change of sensory perception" is meant achieving, at least in part, the ability to recognize and adapt to environmental changes. In terms of sensory hair cell function, a change in sensory perception is associated with the generation of sensory hair cells that convert mechanical stimuli in the inner ear into neural impulses, which are then processed in the brain such that an animal is aware of environmental change, e.g., sound, language, or body/head position. Sensory hair cells are preferably generated in the organ of Corti and/or vestibular apparatus.

Sensory hair cell generation can be determined using a variety of means, such as those known to one skilled in the art. Hair cells can be detected via scanning electron microscopy or via detection of myosin VIIa, a hair cell-specific protein detected by immunochemistry. However, the mere presence of sensory hair cells does not necessarily imply a functional system for recognizing environmental stimuli. Functional sensory hair cells must be operably linked to neural pathways, such that mechanical stimuli are translated to nerve impulses recognized by the brain. Accordingly, while detection of hair cell generation is appropriate for determining successful expression of the atonal-associated nucleic acid sequence to target tissue, examination of subject awareness is a better indicator of changes in sensory perception.

A change in the ability of a subject to detect sound is readily accomplished through administration of simple hearing tests, such as a tone test commonly administered by an audiologist. In most mammals, a reaction to different frequencies indicates a change in sensory perception. In humans, comprehension of language also is appropriate. For example, it is possible for a subject to hear while being unable understand speech. A change in perception is indicated by the ability to distinguish different types of acoustic stimuli, such as differentiating language from background noise, and by understanding speech. Speech threshold and discrimination tests are useful for such evaluations.

Evaluation of changes in balance, motion awareness, and/or timing of response to motion stimuli also is achieved using a variety of techniques. Vestibular function also can be measured by comparing the magnitude of response to motion stimulus (gain) or timing of initiation of response (phase). Animals can be tested for Vestibulo-Ocular Reflex (VOR) gain and phase using scleral search coils to evaluate improvements in sensory perception. Electronystagmography (ENG) records eye movements in response to stimuli such as, for instance, moving or flashing lights, body repositioning, fluid movement inside the semicircular canals, and the like. Evaluation of balance during movement using a rotating chair or moving platform also is useful in this respect.

To detect a change in sensory perception, a baseline value is recorded prior to the inventive method using any appropriate sensory test. A subject is reevaluated at an appropriate time period following the inventive method (e.g., 1 hour, 6 hours, 12 hours, 18 hours, 1 day, 3 days, 5 days, 7 days, 14 days, 21 days, 28 days, 2 months, 3 months or more following the inventive method), the results of which are compared to baseline results to determine a change in sensory perception.

### Method of Treatment

The inventive composition promotes the generation of sensory hair cells that allow perception of stimuli. Ideally, the inventive composition prophylactically or therapeutically treats an animal for at least one disorder associated with loss, damage, absence of sensory hair cells, such as hearing loss and balance disorders. Hearing loss can be caused by damage of hair cells of the organ of Corti due to bacterial or viral infection, heredity, physical injury, acoustic trauma, and the like. While hearing loss is easily identified, balance disorders manifest in a broad variety of complications easily attributable to other ailments. Symptoms of a balance disorder include disorientation, dizziness, vertigo, nausea, blurred vision, clumsiness, and frequent falls. Balance disorders treated by the inventive method preferably involve a peripheral vestibular disorder (i.e., a disturbance in the vestibular apparatus) involving dysfunctional translation of mechanical stimuli into neural impulses due to damage or lack of sensory hair cells.

By "prophylactic" is meant the protection, in whole or in part, against a disorder associated with dysfunctional (or absence of) hair cells, in particular hearing loss or a balance disorder. By "therapeutic" is meant the amelioration of the disorder, itself, and desirably the protection, in whole or in part, against further progression of the disease, e.g., progressive hearing loss. One of ordinary skill in the art will appreciate that any degree of protection from, or amelioration of, a disorder such as hearing loss or balance disruption is beneficial to a patient.

The composition is useful in the treatment of both acute and persistent, progressive disorders associated with lack of or damage to functional sensory hair cells. For acute ailments, an expression vector comprising a nucleic acid sequence encoding an atonal-associated factor (or any hair cell differentiation factor) can be administered using a single application or multiple applications within a short time period. For persistent diseases, such as hearing loss, or disorders stemming from a massive loss of sensory hair cells, numerous rounds of administration of the expression vector may be necessary to realize a therapeutic effect.

### Expression Vectors

One of ordinary skill in the art will appreciate that any of a number of expression vectors known in the art are suitable for introducing the nucleic acid sequence to the inner ear. Examples of suitable expression vectors include, for instance, plasmids, plasmid-liposome complexes, and viral vectors, e.g., parvoviral-based vectors (i.e., adeno-associated virus (AAV)-based vectors), retroviral vectors, herpes simplex virus (HSV)-based vectors, AAV-adenoviral chimeric vectors, and adenovirus-based vectors. Any of these expression vectors can be prepared using standard recombinant DNA techniques described in, e.g., Sambrook et al., Molecular Cloning, A Laboratory Manual, 2d edition, Cold Spring Harbor Press, Cold Spring Harbor, N.Y. (1989), and Ausubel et al., Current Protocols in Molecular Biology, Greene Publishing Associates and John Wiley & Sons, New York, N.Y. (1994).

Plasmids, genetically engineered circular double-stranded DNA molecules, can be designed to contain an expression cassette for delivery of a nucleic acid sequence to the inner ear. Although plasmids were the first vector described for the administration of therapeutic nucleic acids, the level of transfection efficiency is poor compared with other techniques. By complexing the plasmid with liposomes, the efficiency of gene transfer in general is improved. While the liposomes used for plasmid-mediated gene transfer strategies have various compositions, they are typically synthetic cationic lipids. Advantages of plasmid-liposome complexes include their ability to transfer large pieces of DNA encoding a therapeutic nucleic acid and their relatively low immunogenicity. Plasmids also can be modified to prolong transgene expression as described in U.S. Patent 6,165,754. Expression of a transgene in the ear using plasmids has been described (see, for example, Jero et al., Human Gene Therapy, 12, 539-549 (2001)). While plasmids are suitable for use in the inventive method, preferably the expression vector is a viral vector.

AAV vectors are viral vectors of particular interest for use in gene therapy protocols. AAV is a DNA virus, which is not known to cause human disease. AAV requires co-infection with a helper virus (i.e., an adenovirus or a herpes virus), or expression of helper genes, for efficient replication. AAV vectors used for administration of a therapeutic nucleic acid have approximately 96% of the parental genome deleted, such that only the terminal repeats (ITRs), which contain recognition signals for DNA replication and packaging, remain. This eliminates immunologic or toxic side effects due to expression of viral genes. Host cells comprising an integrated AAV genome show no change in cell growth or morphology (see, for example, U.S. Patent 4,797,368). Although efficient, the need for helper virus or helper genes can be an obstacle for widespread use of this vector.

Retrovirus is an RNA virus capable of infecting a wide variety of host cells. Upon infection, the retroviral genome integrates into the genome of its host cell and is replicated along with host cell DNA, thereby constantly producing viral RNA and any nucleic acid sequence incorporated into the retroviral genome. When employing pathogenic retroviruses, e.g., human immunodeficiency virus (HIV) or human T-cell lymphotrophic viruses (HTLV), care must be taken in altering the viral genome to eliminate toxicity. A retroviral vector can additionally be manipulated to render the virus replication-incompetent. As such, retroviral vectors are thought to be particularly useful for stable gene transfer *in vivo.* Lentiviral vectors, such as HIV-based vectors, are exemplary of retroviral vectors used for gene delivery. Unlike other retroviruses, HIV-based vectors are known to incorporate their passenger genes into non-dividing cells and, therefore, are particularly useful in the sensory epithelium of the inner ear where sensory cells do not regenerate.

HSV-based viral vectors are suitable for use as an expression vector to introduce nucleic acids into the inner ear for transduction of target cells. The mature HSV virion consists of an enveloped icosahedral capsid with a viral genome consisting of a linear double-stranded DNA molecule that is 152 kb. Most replication-deficient HSV vectors contain a deletion to remove one or more intermediate-early genes to prevent replication. Advantages of the herpes vector are its ability to enter a latent stage that can result in long-term DNA expression, and its large viral DNA genome that can accommodate exogenous DNA up to 25 kb. Of course, this ability is also a disadvantage in terms of short-term treatment regimens. For a description of HSV-based vectors appropriate for use in the inventive methods, see, for example, U.S. Patents 5,837,532, 5,846,782, 5,849,572, and 5,804,413, and International Patent Applications WO 91/02788, WO 96/04394, WO 98/15637, and WO 99/06583.

Adenovirus (Ad) is a 36 kb double-stranded DNA virus that efficiently transfers DNA *in vivo* to a variety of different target cell types. For use in the inventive method, the virus is preferably made replication-deficient by deleting select genes required for viral replication. The expendable non-replication-essential E3 region is also frequently deleted to allow additional room for a larger DNA insert. The vector can be produced in high titers and can efficiently transfer DNA to replicating and non-replicating cells. Genetic information transferred to a cell by way of an adenoviral vector remains epi-chromosomal, thus eliminating the risks of random insertional mutagenesis and permanent alteration of the genotype of the target cell. However, if desired, the integrative properties of AAV can be conferred to adenovirus by constructing an AAV-Ad chimeric vector. For example, the AAV ITRs and nucleic acid encoding the Rep protein incorporated into an adenoviral vector enables the adenoviral vector to integrate into a mammalian cell genome. Therefore, AAV-Ad chimeric vectors are an interesting option for use in the context of the invention.

The expression vector of the inventive method is a viral vector; namely, the expression vector is an adenoviral vector. Adenoviral serotypes 1 through 51 are available from the American Type Culture Collection (ATCC, Manassas, VA). The adenoviral vector is of subgroup C, namely serotype 5.

The adenoviral vector is replication-deficient. By "replication-deficient" is meant that the adenoviral vector comprises an adenoviral genome that lacks at least one replication-essential gene function (i.e., such that the adenoviral vector does not replicate in typical host cells, especially those in the human patient that could be infected by the adenoviral vector in the course of treatment in accordance with the invention). A deficiency in a gene, gene function, or gene or genomic region, as used herein, is defined as a deletion of sufficient genetic material of the viral genome to impair or obliterate the function of the gene whose nucleic acid sequence was deleted in whole or in part. While deletion of genetic material is preferred, mutation of genetic material by addition or substitute also is appropriate for disrupting gene function. Replication-essential gene functions are those gene functions that are required for replication (e.g., propagation) and are encoded by, for example, the adenoviral early regions (e.g., the E1, E2, and E4 regions), late regions (e.g., the L1-L5 regions), genes involved in viral packaging (e.g., the IVa2 gene), and virus-associated RNAs (e.g., VA-RNA1 and/or VA-RNA-2). More preferably, the replication-deficient adenoviral vector comprises an adenoviral genome deficient in at least one replication-essential gene function of one or more regions of the adenoviral genome. Preferably, the adenoviral vector is deficient in at least one gene function of the E1 region or the E4 region of the adenoviral genome required for viral replication (denoted an E1-deficient adenoviral vector or an E4-deficient adenoviral vector). In addition to a deficiency in the E1 region, the recombinant adenovirus also can have a mutation in the major late promoter (MLP), as discussed in International Patent Application WO 00/00628. Most preferably, the adenoviral vector is deficient in at least one replication-essential gene function (desirably all replication-essential gene functions) of the E1 region and at least part of the nonessential E3 region (e.g., an Xba I deletion of the E3 region) (denoted an E1/E3-deficient adenoviral vector). With respect to the E1 region, the adenoviral vector can be deficient in part or all of the E1A region and part or all of the E1B region, e.g., in at least one replication-essential gene function of each of the E1A and E1B regions. When the adenoviral vector is deficient in at least one replication-essential gene function in one region of the adenoviral genome (e.g., an E1- or E1/E3-deficient adenoviral vector), the adenoviral vector is referred to as "singly replication-deficient."

The adenoviral vector of the invention is "multiply replication-deficient," meaning that the adenoviral vector is deficient in one or more replication-essential gene functions in each of two or more regions of the adenoviral genome. For example, the aforementioned E1-deficient or E1/E3-deficient adenoviral vector can be further deficient in at least one replication-essential gene function of the E4 region (denoted an E1/E4- or E1/E3/E4-deficient adenoviral vector), and/or the E2 region (denoted an E1/E2- or E1/E2/E3-deficient adenoviral vector), preferably the E2A region (denoted an E1/E2A- or E1/E2A/E3-deficient adenoviral vector). Ideally, the adenoviral vector lacks replication-essential gene functions of only those replication-essential gene functions encoded by the early regions of the adenoviral genome, although this is not required in all contexts of the invention. A preferred multiply-deficient adenoviral vector comprises an adenoviral genome having deletions of nucleotides 457-3332 of the E1 region, nucleotides 28593-30470 of the E3 region, nucleotides 32826-35561 of the E4 region, and, optionally, nucleotides 10594-10595 of the region encoding VA-RNA1. However, other deletions may be appropriate. Nucleotides 356-3329 or 356-3510 can be removed to create a deficiency in replication-essential E1 gene functions. Nucleotides 28594-30469 can be deleted from the E3 region of the adenoviral genome. While the specific nucleotide designations recited above correspond to the adenoviral serotype 5 genome, the corresponding nucleotides for non-serotype 5 adenoviral genomes can easily be determined by those of ordinary skill in the art.

The adenoviral vector, when multiply replication-deficient, especially in replication-essential gene functions of the E1 and E4 regions, preferably includes a spacer element to provide viral growth in a complementing cell line similar to that achieved by singly replication-deficient adenoviral vectors, particularly an E1-deficient adenoviral vector. The spacer element can contain any sequence or sequences which are of a desired length, such as sequences at least about 15 base pairs (e.g., between about 15 base pairs and about 12,000 base pairs), preferably about 100 base pairs to about 10,000 base pairs, more preferably about 500 base pairs to about 8,000 base pairs, even more preferably about 1,500 base pairs to about 6,000 base pairs, and most preferably about 2,000 to about 3,000 base pairs in length. The spacer element sequence can be coding or non-coding and native or non-native with respect to the adenoviral genome, but does not restore the replication-essential function to the deficient region. The use of a spacer in an adenoviral vector is described in U.S. Patent 5,851,806. In one embodiment of the inventive method, the replication-deficient or conditionally-replicating adenoviral vector is an E1/E4-deficient adenoviral vector wherein the L5 fiber region is retained, and a spacer is located between the L5 fiber region and the right-side ITR. More preferably, in such an adenoviral vector, the E4 polyadenylation sequence alone or, most preferably, in combination with another sequence, exists between the L5 fiber region and the right-side ITR, so as to sufficiently separate the retained L5 fiber region from the right-side ITR, such that viral production of such a vector approaches that of a singly replication-deficient adenoviral vector, particularly an E1-deficient adenoviral vector.

The adenoviral vector can be deficient in replication-essential gene functions of only the early regions of the adenoviral genome, only the late regions of the adenoviral genome, and both the early and late regions of the adenoviral genome. The adenoviral vector also can have essentially the entire adenoviral genome removed, in which case it is preferred that at least either the viral inverted terminal repeats (ITRs) and one or more promoters or the viral ITRs and a packaging signal are left intact (i.e., an adenoviral amplicon). The 5' or 3' regions of the adenoviral genome comprising ITRs and packaging sequence need not originate from the same adenoviral serotype as the remainder of the viral genome. For example, the 5' region of an adenoviral serotype 5 genome (i.e., the region of the genome 5' to the adenoviral E1 region) can be replaced with the corresponding region of an adenoviral serotype 2 genome (e.g., the Ad5 genome region 5' to the E1 region of the adenoviral genome is replaced with nucleotides 1-456 of the Ad2 genome). Suitable replication-deficient adenoviral vectors, including multiply replication-deficient adenoviral vectors, are disclosed in U.S. Patents 5,837,511, 5,851,806, and 5,994,106, U.S. Published Patent Applications 2001/0043922 A1 2002/0004040 A1, 2002/0031831 A1, and 2002/0110545 A1, and International Patent Applications WO 95/34671, WO 97/12986, and WO 97/21826. Ideally, the replication-deficient adenoviral vector is present in a pharmaceutical composition virtually free of replication-competent adenovirus (RCA) contamination (e.g., the pharmaceutical composition comprises less than about 1% of RCA contamination). Most desirably, the pharmaceutical composition is RCA-free. Adenoviral vector compositions and stocks that are RCA-free are described in U.S. Patent 5,944,106 and 6,482,616, U.S. Published Patent Application 2002/0110545 A1, and International Patent Application WO 95/34671.

Therefore, the expression vector of the inventive composition is a multiply replication-deficient adenoviral vector lacking all or part of the E1 region, all or part of the E3 region, all or part of the E4 region, and, optionally, all or part of the E2 region. It is believed that multiply deficient vectors are particularly suited for delivery of exogenous nucleic acid sequences to the ear. Adenoviral vectors deficient in at least one replication-essential gene function of the E1 region are most commonly used for gene transfer *in vivo.* However, currently used singly replication-deficient adenoviral vectors can be detrimental to the sensitive cells of the epithelium of the inner ear, causing damage to the very cells to be treated. Adenoviral vectors that are deficient in at least one replication-essential gene function of the E4 region, particularly adenoviral vectors deficient in replication-essential gene functions of the E4 region and the E1 region, are less toxic to cells than E1-deficient adenoviral vectors (see, for example, Wang et al., Nature Medicine, 2(6), 714-716 (1996) and U.S. Patent 6,228,646). Accordingly, damage to existing hair cells and supporting cells can be minimized by employing an E1,E4-deficient adenoviral vector to deliver the nucleic acid sequence encoding the atonal-associated factor to inner ear cells.

In this regard, it has been observed that an at least E4-deficient adenoviral vector expresses a transgene at high levels for a limited amount of time *in vivo* and that persistence of expression of a transgene in an at least E4-deficient adenoviral vector can be modulated through the action of a trans-acting factor, such as HSV ICP0, Ad pTP, CMV-IE2, CMV-IE86, HIV tat, HTLV-tax, HBV-X, AAV Rep 78, the cellular factor from the U205 osteosarcoma cell line that functions like HSV ICP0, or the cellular factor in PC12 cells that is induced by nerve growth factor, among others. In view of the above, the multiply deficient adenoviral vector (e.g., the at least E4-deficient adenoviral vector) or a second expression vector comprises a nucleic acid sequence encoding a trans-acting factor that modulates the persistence of expression of the nucleic acid sequence encoding the atonal-associated factor, as described in, for example, U.S. Patent 6,225,113, 6,660,521, and 6,649,373, and International Patent Application WO 00/34496.

Replication-deficient adenoviral vectors are typically produced in complementing cell lines that provide gene functions not present in the replication-deficient adenoviral vectors, but required for viral propagation, at appropriate levels in order to generate high titers of viral vector stock. A preferred cell line complements for at least one and preferably all replication-essential gene functions not present in a replication-deficient adenovirus. The complementing cell line can complement for a deficiency in at least one replication-essential gene function encoded by the early regions, late regions, viral packaging regions, virus-associated RNA regions, or combinations thereof, including all adenoviral functions (e.g., to enable propagation of adenoviral amplicons). Most preferably, the complementing cell line complements for a deficiency in at least one replication-essential gene function (e.g., two or more replication-essential gene functions) of the E1 region of the adenoviral genome, particularly a deficiency in a replication-essential gene function of each of the E1A and E1B regions. In addition, the complementing cell line can complement for a deficiency in at least one replication-essential gene function of the E2 (particularly as concerns the adenoviral DNA polymerase and terminal protein) and/or E4 regions of the adenoviral genome. Desirably, a cell that complements for a deficiency in the E4 region comprises the E4-ORF6 gene sequence and produces the E4-ORF6 protein. Such a cell desirably comprises at least ORF6 and no other ORF of the E4 region of the adenoviral genome. The cell line preferably is further characterized in that it contains the complementing genes in a non-overlapping fashion with the adenoviral vector, which minimizes, and practically eliminates, the possibility of the vector genome recombining with the cellular DNA. Accordingly, the presence of replication competent adenoviruses (RCA) is minimized if not avoided in the vector stock, which, therefore, is suitable for certain therapeutic purposes, especially gene therapy purposes. The lack of RCA in the vector stock avoids the replication of the adenoviral vector in non-complementing cells. Construction of such a complementing cell lines involve standard molecular biology and cell culture techniques, such as those described by Sambrook et al., Molecular Cloning, a Laboratory Manual, 2d edition, Cold Spring Harbor Press, Cold Spring Harbor, N.Y. (1989), and Ausubel et al., Current Protocols in Molecular Biology, Greene Publishing Associates and John Wiley & Sons, New York, N.Y. (1994).

Complementing cell lines for producing the adenoviral vector include, but are not limited to, 293 cells (described in, e.g., Graham et al., J. Gen. Virol., 36, 59-72 (1977)), PER.C6 cells (described in, e.g., International Patent Application WO 97/00326, and U.S. Patents 5,994,128 and 6,033,908), and 293-ORF6 cells (described in, e.g., International Patent Application WO 95/34671 and Brough et al., J. Virol., 71, 9206-9213 (1997)). In some instances, the complementing cell will not complement for all required adenoviral gene functions. Helper viruses can be employed to provide the gene functions in *trans* that are not encoded by the cellular or adenoviral genomes to enable replication of the adenoviral vector. Adenoviral vectors can be constructed, propagated, and/or purified using the materials and methods set forth, for example, in U.S. Patents 5,965,358, 5,994,128, 6,033,908, 6,168,941, 6,329,200, 6,383,795, 6,440,728, 6,447,995, and 6,475,757, U.S. Patent Application Publication No. 2002/0034735 A1, and International Patent Applications WO 98/53087, WO 98/56937, WO 99/15686, WO 99/54441, WO 00/12765, WO 01/77304, and WO 02/29388, as well as the other references identified herein. Non-group C adenoviral vectors, including adenoviral serotype 35 vectors, can be produced using the methods set forth in, for example, U.S. Patents 5,837,511 and 5,849,561, and International Patent Applications WO 97/12986 and WO 98/53087. Moreover, numerous adenoviral vectors are available commercially.

The adenoviral vector's coat protein can be modified so as to decrease the adenoviral vector's ability or inability to be recognized by a neutralizing antibody directed against the wild-type coat protein. Such modifications are useful for multiple rounds of administration. Similarly, the coat protein of the adenoviral vector can be manipulated to alter the binding specificity or recognition of the adenoviral vector for a viral receptor on a potential host cell. Such manipulations can include deletion or substitution of regions of the fiber, penton, hexon, pllla, pVI, and/or pIX, insertions of various native or non-native ligands into portions of the coat protein, and the like. Manipulation of the coat protein can broaden the range of cells infected by the adenoviral vector or enable targeting of the adenoviral vector to a specific cell type. The ability of an adenoviral vector to recognize a potential host cell can be modulated without genetic manipulation of the coat protein, i.e., through use of a bi-specific molecule. For instance, complexing an adenovirus with a bispecific molecule comprising a penton base- or fiber-binding domain and a domain that selectively binds a particular cell surface binding site enables the targeting of the adenoviral vector to a particular cell type.

Preferably, the adenoviral capsid is modified to display a non-native amino acid sequence. The non-native amino acid sequence can be inserted into or in place of an internal coat protein sequence (e.g., within an exposed loop of an adenoviral fiber protein) or fused to the terminus of an adenoviral coat protein (e.g., fused to the C-terminus of an adenoviral fiber protein, optionally using a linker or spacer sequence). The non-native amino acid sequence can be conjugated to any of the adenoviral coat proteins to form a chimeric coat protein. Therefore, for example, the non-native amino acid sequence of the invention can be conjugated to, inserted into, or attached to a fiber protein, a penton base protein, a hexon protein, proteins IX, VI, or IIIa, etc. The sequences of such proteins, and methods for employing them in recombinant proteins, are well known in the art (see, e.g., U.S. Patents 5,543,328; 5,559,099; 5,712,136; 5,731,190; 5,756,086; 5,770,442; 5,846,782; 5,962,311; 5,965,541; 5,846,782; 6,057,155; 6,127,525; 6,153,435; 6,329,190; 6,455,314; 6,465,253; and 6,576,456; U.S. Patent Application Publication 2001/0047081 and 2003/0099619; and International Patent Applications WO 96/07734, WO 96/26281, WO 97/20051, WO 98/07877, WO 98/07865, WO 98/40509, WO 98/54346, WO 00/15823, WO 01/58940, and WO 01/92549). The coat protein portion of the chimeric coat protein can be a full-length adenoviral coat protein to which the ligand domain is appended, or it can be truncated, e.g., internally or at the C- and/or N- terminus. The coat protein portion need not, itself, be native to the adenoviral vector.

Where the ligand is attached to the fiber protein, preferably it does not disturb the interaction between viral proteins or fiber monomers. Thus, the non-native amino acid sequence preferably is not itself an oligomerization domain, as such can adversely interact with the trimerization domain of the adenovirus fiber. Preferably the ligand is added to the virion protein, and is incorporated in such a manner as to be readily exposed to the substrate (e.g., at the N- or C- terminus of the protein, attached to a residue facing the substrate, positioned on a peptide spacer to contact the substrate, etc.) to maximally present the non-native amino acid sequence to the substrate. Ideally, the non-native amino acid sequence is incorporated into an adenoviral fiber protein at the C-terminus of the fiber protein (and attached via a spacer) or incorporated into an exposed loop (e.g., the HI loop) of the fiber to create a chimeric coat protein. Where the non-native amino acid sequence is attached to or replaces a portion of the penton base, preferably it is within the hypervariable regions to ensure that it contacts the substrate. Where the non-native amino acid sequence is attached to the hexon, preferably it is within a hypervariable region (Miksza et al., J. Virol., 70(3), 1836-44 (1996)). Use of a spacer sequence to extend the non-native amino acid sequence away from the surface of the adenoviral particle can be advantageous in that the non-native amino acid sequence can be more available for binding to a receptor and any steric interactions between the non-native amino acid sequence and the adenoviral fiber monomers is reduced.

A chimeric viral coat protein comprising a non-native ligand is desirably able to direct entry into cells of the viral, i.e., adenoviral, vector comprising the coat protein that is more efficient than entry into cells of a vector that is identical except for comprising a wild-type viral coat protein rather than the chimeric viral coat protein. Preferably, the chimeric virus coat protein binds a novel endogenous binding site present on the cell surface that is not recognized, or is poorly recognized by a vector comprising a wild-type coat protein.

In addition, the adenoviral capsid proteins can be altered to reduce or ablate binding to native adenoviral receptors (i.e., receptors bound by wild-type adenovirus). In particular, the portion of the adenoviral fiber protein which interacts with the coxsackie and adenovirus receptor (CAR) can be mutated by deletion, substitution, repositioning within the fiber protein, etc., such that the adenoviral fiber protein does not bind CAR. Likewise, the portion of the adenoviral penton protein that interacts with integrins can be altered to ablate native integrin binding. To reduce native binding and transduction of the replication-deficient or conditionally-replicating adenoviral vector, the native binding sites located on adenoviral coat proteins which mediate cell entry, e.g., the fiber and/or penton base, are absent or disrupted. Two or more of the adenoviral coat proteins are believed to mediate attachment to cell surfaces (e.g., the fiber and penton base). Any suitable technique for altering native binding to a host cell (e.g., a mesothelial cell or hepatocyte) can be employed. For example, exploiting differing fiber lengths to ablate native binding to cells can be accomplished via the addition of a binding sequence to the penton base or fiber knob. This addition can be done either directly or indirectly via a bispecific or multispecific binding sequence. Alternatively, the adenoviral fiber protein can be modified to reduce the number of amino acids in the fiber shaft, thereby creating a "short-shafted" fiber (as described in, for example, U.S. Patent 5,962,311). The fiber proteins of some adenoviral serotypes are naturally shorter than others, and these fiber proteins can be used in place of the native fiber protein to reduce native binding of the adenovirus to its native receptor. For example, the native fiber protein of an adenoviral vector derived from serotype 5 adenovirus can be switched with the fiber protein from adenovirus serotypes 40 or 41.

In this regard, the adenoviral vector can be modified to include an adenoviral coat protein (e.g., fiber, penton, or hexon protein) from a different serotype of adenovirus. For example, an adenoviral serotype 5 adenovirus can be modified to display an adenovirus serotype 35 fiber, which, in turn, can optionally comprise one or more non-native amino acid ligands. It is possible to utilize an adenoviral vector which does not naturally infect cell types of the inner ear to target the vector to a particular cell type. Alternatively, an adenoviral vector which naturally transduces cells of the inner ear can be modified to display an adenoviral fiber protein and/or adenoviral penton base derived from an adenovirus which has no natural tropism for target cells, which adenoviral vector can display a non-native amino acid sequence that enables transduction of target cells.

In another embodiment, the nucleic acid residues associated with native substrate binding can be mutated (see, e.g., International Patent Application WO 00/15823; Einfeld et al., J. Virol., 75(23), 11284-11291 (2001); and van Beusechem et al., J. Virol., 76(6), 2753-2762 (2002)) such that the adenoviral vector incorporating the mutated nucleic acid residues is less able to bind its native substrate. For example, adenovirus serotypes 2 and 5 transduce cells via binding of the adenoviral fiber protein to the coxsackievirus and adenovirus receptor (CAR) and binding of penton proteins to integrins located on the cell surface. Accordingly, the replication-deficient or conditionally-replicating adenoviral vector of the inventive method can lack native binding to CAR and/or exhibit reduced native binding to integrins. To reduce native binding of the replication-deficient or conditionally-replicating adenoviral vector to host cells, the native CAR and/or integrin binding sites (e.g., the RGD sequence located in the adenoviral penton base) are removed or disrupted.

Modifications to adenoviral coat proteins can enhance the resulting adenoviral vectors' ability to evade the host immune system. In one embodiment, the adenoviral vector is selectively targeted to scarred epithelial cells (e.g., regions of the epithelium missing endogenous, functional hair cells) by ablation of native binding of the adenoviral vector to CAR and/or integrins and incorporation into the adenoviral capsid one or more non-native ligands. Suitable ligands that mediate transduction via a specific receptor can be determined using routine library display techniques (such as phage display) and include, for example, ligands bound by EGF and ligands from the FGF family of peptides. Other examples of non-native amino acid sequences and their substrates include, but are not limited to, short (e.g., 6 amino acids or less) linear stretches of amino acids recognized by integrins, as well as polyamino acid sequences such as polylysine, polyarginine, etc. Non-native amino acid sequences for generating chimeric adenoviral coat proteins are further described in U.S. Patent 6,455,314 and International Patent Application WO 01/92549.

Suitable modifications to an adenoviral vector are described in U.S. Patents 5,543,328, 5,559,099, 5,712,136, 5,731,190, 5,756,086, 5,770,442, 5,846,782, 5,871,727, 5,885,808, 5,922,315, 5,962,311, 5,965,541, 6,057,155, 6,127,525, 6,153,435, 6,329,190, 6,455,314, and 6,465,253, U.S. Published Applications 2001/0047081 A1, 2002/0099024 A1, and 2002/0151027 A1, and International Patent Applications WO 96/07734, WO 96/26281, WO 97/20051, WO 98/07865, WO 98/07877, WO 98/40509, WO 98/54346, WO 00/15823, WO 01/58940, and WO 01/92549. The construction of adenoviral vectors is well understood in the art. Adenoviral vectors can be constructed and/or purified using the methods set forth, for example, in U.S. Patents 5,965,358, 6,168,941, 6,329,200, 6,383,795, 6,440,728, 6,447,995, and 6,475,757, and International Patent Applications WO 98/53087, WO 98/56937, WO 99/15686, WO 99/54441, WO 00/12765, WO 01/77304, and WO 02/29388, as well as the other references identified herein. Moreover, numerous expression vectors, including adenoviral vectors, are available commercially. Adeno-associated viral vectors can be constructed and/or purified using the methods set forth, for example, in U.S. Patent 4,797,368 and Laughlin et al., Gene, 23, 65-73 (1983).

The selection of an expression vector for use in the inventive method depends on a variety of factors such as, for example, the host, immunogenicity of the vector, the desired duration of protein production, the target cell, and the like. As each type of expression vector has distinct properties, the inventive method can be tailored to any particular situation. Moreover, more than one type of expression vector can be used to deliver the nucleic acid sequence to the target cell. Thus, the invention provides a method of changing the sensory perception of an animal, wherein the method comprises administering to the inner ear at least two different expression vectors, each comprising a nucleic acid sequence encoding an atonal-associated factor and/or a nucleic acid sequence encoding at least one other therapeutic agent, such as a neurotrophic agent. Preferably, the target cell in the inner ear, e.g., a supporting cell, is contacted with an adenoviral vector and an HSV vector, in that adenoviral vectors efficiently transduce supporting cells and HSV vectors efficiently transduce neurons. One of ordinary skill in the art will appreciate the ability to capitalize on the advantageous properties of multiple delivery systems to treat or study sensory disorders of the inner ear.

### Nucleic Acid Sequence Encoding An Atonal-Associated Factor

The expression vector of the inventive composition comprises a nucleic acid encoding an atonal-associated factor. Atonal-associated factors are a family of transcription factors that transdifferentiate supporting cells into sensory hair cells in the ear. Atonal-associated factors are transcription factors of the basic helix-loop-helix (bHLH) family of proteins. The basic domain of the protein is responsible for DNA binding and function of the protein. The *Drosophila* bHLH protein (*ato*) activates genes associated with the development of sensory organs of the insect, specifically chordotonal organs. Atonal-associated factors are found in a variety of animals and insects, including mice (mouse atonal homolog 1 (*Math1*)), chickens (chicken atonal homolog 1 (*Cath1*)), *Xenopus (Xenopus* atonal homolog 1 (*Xath1*)), and humans (human atonal homolog 1 (*Hath1*)). *Math1* is highly homologous to *ato* in the bHLH domain (82% amino acid similarity) with 100% conservation of the basic domain, and functions in determining cell fate in mice. Math1 has been shown to be essential for hair cell development and can stimulate hair cell regeneration in the ear. Math1 is further characterized in, for example, Ben-Arie et al., Human Molecular Genetics, 5, 1207-1216 (1996), Bermingham et al., Science, 284, 1837-1841 (1999), Zheng and Gao, Nature Neuroscience, 3(2), 580-586 (2000), Chen et al., Development, 129, 2495-2505 (2002). Hathl is the human counterpart of Math1. Accordingly, an atonal-associated factor is a peptide that promotes differentiation of supporting cells into sensory hair cells, and comprises an amino acid having a sequence significantly similar to that of Math1 and Hath1. Atonal-associated factors are further described in International Patent Application WO 00/73764. According to the invention, the atonal-associated factor is Hath1.

Thus, the atonal-associated factor encoded by the nucleic acid sequence of the invention is a protein or peptide comprising an amino acid sequence having at least about 50% sequence identity to the amino acid sequence of Math1 (SEQ ID NO: 1 and 2, and GenBank Accession No. BAA07791, GI No. 994771), and having the ability to transdifferentiate supporting cells into sensory hair cells. Ideally, at least about 60% homology (e.g., at least about 65%, or at least about 70%, sequence identity), preferably at least about 75% sequence identity (e.g., at least about 80%, or at least about 85%, sequence identity), and most preferably at least about 90% sequence identity (e.g., at least about 95% sequence identity) exists compared to the Math1 amino acid sequence. The Math1 and Hath1 amino acid sequences are significantly similar and, as such, the atonal-associated factor can comprise at least about 50% sequence identity (e.g., at least about 60%, at least about 65%, at least about 70%, at least about 75%, at least about 80%, at least about 85%, at least about 90%, or at least about 95% sequence identity) to the amino acid sequence of Hath1 (SEQ ID NO: 3 and 4, and GenBank Accession No. AAB41305.1, GI No. 1575355, and disclosed in WO 00/73764), and having the ability to transdifferentiate supporting cells into sensory hair cells. Looking to the nucleic acid sequence, preferably the nucleic acid sequence encoding an atonal associated factor is the coding sequence of the *Math1* gene or *Hath1* gene (i.e., the portion of the *Math1* or *Hath1* genes that encode the Math1 and Hath 1 proteins absent the regulatory sequences associated with the gene) or cDNA encoding the Math1 or Hath1 protein. Nucleic acid sequences encoding Math1 and Hath1 are provided herein as SEQ ID NOS: 6 and 7 and are publicly available as GenBank Accession Nos. D43694 (GI No. 994770) and U61148 (GI No. 1575354).

While the nucleic acid sequence encoding the atonal-associated factor preferably is that described in International Patent Application WO 00/73764, many modifications and variations (e.g., mutation) of the nucleic acid sequence are possible and appropriate in the context of the invention. For example, the degeneracy of the genetic code allows for the substitution of nucleotides throughout the coding sequence, as well as in the translational stop signal, without alteration of the encoded polypeptide. Such substitutable sequences can be deduced from the known amino acid sequence of an atonal-associated factor or nucleic acid sequence encoding an atonal-associated factor and can be constructed by conventional synthetic or site-specific mutagenesis procedures. Synthetic DNA methods can be carried out in substantial accordance with the procedures of Itakura et al., Science, 198, 1056-1063 (1977), and Crea et al., Proc. Natl. Acad. Sci. USA, 75, 5765-5769 (1978). Site-specific mutagenesis procedures are described in Maniatis et al., Molecular Cloning: A Laboratory Manual, Cold Spring Harbor, NY (2d ed. 1989). Alternatively, the nucleic acid sequence can encode an atonal-associated peptide with extensions on either the Nor C-terminus of the protein, so long as the resulting atonal-associated factor retains activity (i.e., the ability to transdifferentiate supporting cells into sensory hair cells).

It is believed that the function of atonal-associated factors is dependent on the helix-loop-helix (HLH) portion of the protein, particularly the basic region of the HLH domain (Chien et al., Proc. Natl. Acad. Sci., 93, 13239-13244 (1996)). Accordingly, any modification of the atonal-associated factor amino acid sequence desirably is located outside of the basic domain of the protein such that the amino acid sequence of the basic domain has at least about 50% sequence identity (e.g., at least about 55%, at least about 60%, or at least about 65% sequence identity) to the HLH domain of the Hath1 amino acid sequence. Preferably, the atonal-associated factor or mutant or fragment thereof has at least about 75% sequence identity, more preferably at least about 85% sequence identity, even more preferably at least about 90% sequence identity (e.g., at least about 95% sequence identity) to the HLH domain of the Hath1 amino acid sequence. Also desirably, any modification of the atonal-associated factor amino acid sequence desirably is located outside of the basic domain of the protein such that the amino acid sequence of the basic domain has at least about 50% sequence identity (e.g., at least about 55%, at least about 60%, or at least about 65% sequence identity), preferably at least about 70% sequence identity (e.g., at least about 75%, at least about 80%, or at least about 85% sequence identity), more preferably at least about 90% sequence identity (e.g., at least about 95% sequence identity and preferably 100% identity) to the basic domain of the Hath1 amino acid sequence. Also preferably, the amino acid sequence of the atonal-associated factor comprises a region having at least about 75% identity to (e.g., at least about 80% identity to, at least about 85% identity to, at least about 90% identity to, or at least about 95% identity to) AANARERRRMHGLNHAFDQLR (SEQ ID NO: 5), which comprises the basic region of the atonal-associated factor and the first helix region of the helix-loop-helix motif (Jarman et al., Cell, 79, 1307-1321 (1993)).

The expression vector, e.g., the viral vector (preferably the adenoviral or the adeno-associated viral vector), also can comprise a nucleic acid sequence encoding a therapeutic fragment of an atonal-associated factor. One of ordinary skill in the art will appreciate that any transcription factor, e.g., Math1 or Hath1, can be modified or truncated and retain transcription activating activity. As such, therapeutic fragments (i.e., those fragments having biological activity sufficient to, for example, activate transcription) also are suitable for incorporation into the expression vector. Likewise, a fusion protein comprising an atonal-associated factor or a therapeutic fragment thereof and, for example, a moiety that stabilizes peptide conformation, also can be present in the expression vector. A functioning atonal-associated factor or therapeutic fragment thereof transdifferentiates supporting cells into sensory hair cells, thereby desirably effecting a change in an animal's ability to perceive environmental stimuli.

The degree of amino acid identity can be determined using any method known in the art, such as the BLAST sequence database. Furthermore, a homolog of the protein can be any peptide, polypeptide, or portion thereof, which hybridizes to the protein under at least moderate, preferably high, stringency conditions, and retains activity. Exemplary moderate stringency conditions include overnight incubation at 37 °C in a solution comprising 20% formamide, 5x SSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1x SSC at about 37-50° C, or substantially similar conditions, e.g., the moderately stringent conditions described in Sambrook et al., *supra.* High stringency conditions are conditions that use, for example (1) low ionic strength and high temperature for washing, such as 0.015 M sodium chloride/0.0015 M sodium citrate/0.1% sodium dodecyl sulfate (SDS) at 50° C, (2) employ a denaturing agent during hybridization, such as formamide, for example, 50% (v/v) formamide with 0.1% bovine serum albumin (BSA)/0.1% Ficoll/0.1% polyvinylpyrrolidone (PVP)/50 mM sodium phosphate buffer at pH 6.5 with 750 mM sodium chloride, 75 mM sodium citrate at 42° C, or (3) employ 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42° C, with washes at (i) 42° C in 0.2x SSC, (ii) at 55° C in 50% formamide and (iii) at 55° C in 0.1x SSC (preferably in combination with EDTA). Additional details and an explanation of stringency of hybridization reactions are provided in, e.g., Ausubel et al., *supra.*

The nucleic acid sequence is desirably present as part of an expression cassette, i.e., a particular base sequence that possesses functions which facilitate subcloning and recovery of a nucleic acid sequence (e.g., one or more restriction sites) or expression of a nucleic acid sequence (e.g., polyadenylation or splice sites). When the expression vector is an adenoviral vector, the nucleic acid sequence coding for an atonal-associated factor can be located in the E1 region (e.g., replaces the E1 region in whole or in part) or can be located in the E4 region of the adenoviral genome. When positioned in the E4 region, a spacer sequence is not required. The expression cassette is preferably inserted in a 3'-5' orientation, e.g., oriented such that the direction of transcription of the expression cassette is opposite that of the surrounding adenoviral genome. In addition to the expression cassette comprising the nucleic acid sequence encoding an atonal-associated factor, the adenoviral vector can comprise other expression cassettes containing nucleic acid sequences encoding other gene products, which cassettes can replace any of the deleted regions of the adenoviral genome. The insertion of an expression cassette into the adenoviral genome (e.g., the E1 region of the genome) can be facilitated by known methods, for example, by the introduction of a unique restriction site at a given position of the adenoviral genome. As set forth above, preferably the E3 region of the adenoviral vector is deleted, and the E4 region is replaced by a spacer element.

The nucleic acid sequence is operably linked to regulatory sequences necessary for expression, e.g., a promoter. A "promoter" is a DNA sequence that directs the binding of RNA polymerase and thereby promotes RNA synthesis. A nucleic acid sequence is "operably linked" to a promoter when the promoter is capable of directing transcription of that nucleic acid sequence. A promoter can be native or non-native to the nucleic acid sequence to which it is operably linked. Any promoter (i.e., whether isolated from nature or produced by recombinant DNA or synthetic techniques) can be used in connection with the invention to provide for transcription of the nucleic acid sequence. The promoter preferably is capable of directing transcription in a eukaryotic (desirably mammalian) cell. The functioning of the promoter can be altered by the presence of one or more enhancers (e.g., the CMV immediate early enhancer) and/or silencers.

The invention preferentially employs a viral promoter. Suitable viral promoters are known in the art and include, for instance, cytomegalovirus (CMV) promoters, such as the CMV immediate-early promoter, promoters derived from human immunodeficiency virus (HIV), such as the HIV long terminal repeat promoter, Rous sarcoma virus (RSV) promoters, such as the RSV long terminal repeat, mouse mammary tumor virus (MMTV) promoters, HSV promoters, such as the Lap2 promoter or the herpes thymidine kinase promoter (Wagner et al., Proc. Natl. Acad. Sci., 78, 144-145 (1981)), promoters derived from SV40 or Epstein Barr virus, an adeno-associated viral promoter, such as the p5 promoter, and the like. Preferably, the viral promoter is an adenoviral promoter, such as the Ad2 or Ad5 major late promoter and tripartite leader, a CMV promoter (murine or human in origin), or an RSV promoter.

The promoter need not be a viral promoter. For example, the promoter can be a cellular promoter, i.e., a promoter that drives expression of a cellular protein. Preferred cellular promoters for use in the invention will depend on the desired expression profile to produce the therapeutic agent(s). In one aspect, the cellular promoter is preferably a constitutive promoter that works in a variety of cell types. Suitable constitutive promoters can drive expression of genes encoding transcription factors, housekeeping genes, or structural genes common to eukaryotic cells. For example, the Ying Yang 1 (YY1) transcription factor (also referred to as NMP-1, NF-E1, and UCRBP) is a ubiquitous nuclear transcription factor that is an intrinsic component of the nuclear matrix (Guo et al., PNAS, 92, 10526-10530 (1995)). JEM-1 (also known as HGMW and BLZF-1; Tong et al., Leukemia, 12(11), 1733-1740 (1998), and Tong et al., Genomics, 69(3), 380-390 (2000)), a ubiquitin promoter, specifically UbC (Marinovic et al., J. Biol. Chem., 277(19), 16673-16681 (2002)), a b-actin promoter, such as that derived from chicken, and the like are appropriate for use in the inventive method.

Many of the above-described promoters are constitutive promoters. Instead of being a constitutive promoter, the promoter can be an inducible promoter, i.e., a promoter that is up- and/or down-regulated in response to appropriate signals. For instance, suitable inducible promoter systems include, but are not limited to, the IL-8 promoter, the metallothionine inducible promoter system, the bacterial *lacZYA* expression system, the tetracycline expression system, and the T7 polymerase system. Further, promoters that are selectively activated at different developmental stages (e.g., globin genes are differentially transcribed from globin-associated promoters in embryos and adults) can be employed. The promoter sequence that regulates expression of the nucleic acid sequence can contain at least one heterologous regulatory sequence responsive to regulation by an exogenous agent. The regulatory sequences are preferably responsive to exogenous agents such as, but not limited to, drugs, hormones, or other gene products. For example, the regulatory sequences, e.g., promoter, preferably are responsive to glucocorticoid receptor-hormone complexes, which, in turn, enhance the level of transcription of a therapeutic peptide or a therapeutic fragment thereof.

Preferably, the regulatory sequences comprise a tissue-specific promoter, i.e., a promoter that is preferentially activated in a given tissue and results in expression of a gene product in the tissue where activated. A tissue specific promoter for use in the inventive vector can be chosen by the ordinarily skilled artisan based upon the target tissue or cell-type. Suitable promoters include, but are not limited to, BRN.3C, BRN 3.1, the POU ORF3 factor promoter, BRK1, BRK3, the chordin promoter, the noggin promoter, the jagged 1 promoter, the jagged2 promoter, and the notch1 promoter. Preferred tissue-specific promoters for use in the inventive method are specific to supporting cells or sensory hair cells, such as an atonal promoter or a myosin VIIa promoter, which function in hair cells, or a hes-1 promoter, which functions in supporting cells. Ideally, a promoter is selected that promotes transgene expression in scarred epithelium.

A promoter also can be selected for use in the method of the invention by matching its particular pattern of activity with the desired pattern and level of expression of the desired protein (e.g., the atonal-associated factor). Alternatively, a hybrid promoter can be constructed which combines the desirable aspects of multiple promoters. For example, a CMV-RSV hybrid promoter combining the CMV promoter's initial rush of activity with the RSV promoter's high maintenance level of activity is especially preferred for use in many embodiments of the inventive method. It is also possible to select a promoter with an expression profile that can be manipulated by an investigator.

Along these lines, to optimize protein production, preferably the nucleic acid sequence further comprises a polyadenylation site following the coding region of the nucleic acid sequence. Any suitable polyadenylation sequence can be used, including a synthetic optimized sequence, as well as the polyadenylation sequence of BGH (Bovine Growth Hormone), polyoma virus, TK (Thymidine Kinase), EBV (Epstein Barr Virus), and the papillomaviruses, including human papillomaviruses and BPV (Bovine Papilloma Virus). A preferred polyadenylation sequence is the SV40 (Human Sarcoma Virus-40) polyadenylation sequence. Also, preferably all the proper transcription signals (and translation signals, where appropriate) are correctly arranged such that the nucleic acid sequence is properly expressed in the cells into which it is introduced. If desired, the nucleic acid sequence also can incorporate splice sites (i.e., splice acceptor and splice donor sites) to facilitate mRNA production. Moreover, if the nucleic acid sequence encodes a protein or peptide, which is a processed or secreted protein or acts intracellularly, preferably the nucleic acid sequence further comprises the appropriate sequences for processing, secretion, intracellular localization, and the like.

In certain embodiments, it may be advantageous to modulate expression of the atonal-associated factor. An especially preferred method of modulating expression of a nucleic acid sequence comprises addition of site-specific recombination sites on the expression vector. Contacting an expression vector comprising site-specific recombination sites with a recombinase will either up- or down-regulate transcription of a coding sequence, or simultaneously up-regulate transcription of one coding sequence and down-regulate transcription of another, through the recombination event. Use of site-specific recombination to modulate transcription of a nucleic acid sequence is described in, for example, U.S. Patents 5,801,030 and 6,063,627 and International Patent Application WO 97/09439.

In view of the above, the invention further provides an adenoviral vector comprising a nucleic acid sequence encoding an atonal-associated factor (e.g., Math1 or Hath1) or a therapeutic fragment thereof, wherein the nucleic acid sequence is operably linked to regulatory sequences necessary for expression of the atonal-associated factor or a therapeutic fragment thereof. The adenoviral vector is deficient in at least one replication-essential gene function of at least the E4 region. The nucleic acid sequence can be obtained from any source, e.g., isolated from nature, synthetically generated, isolated from a genetically engineered organism, and the like. Appropriate adenoviral vectors and regulatory sequences are discussed herein. For example, the invention further provides a method of generating a hair cell in differentiated sensory epithelia *in vivo.* The method comprises contacting differentiated sensory epithelial cells with an adenoviral vector (a) deficient in one or more replication-essential gene functions of the E1 region, the E4 region, and, optionally, one or more gene functions the E3 region, (b) comprising a spacer in the E4 region, and (c) comprising a nucleic acid sequence encoding an atonal-associated factor. The nucleic acid sequence is expressed to produce the atonal-associated factor such that a hair cell is generated. While the adenoviral vector can be used to generate hair cells *in vivo* (and therefore is useful for prophylactically or therapeutically treat a hearing disorder or a balance disorder), transdifferentiation of supporting cells can occur *in vitro* and, thus, can be used in methods of research.

### Routes of Administration

One skilled in the art will appreciate that suitable methods of administering an expression vector, such as an adenoviral vector, to the inner ear are available. Although more than one route can be used to administer a particular expression vector, a particular route can provide a more immediate and more effective reaction than another route. Accordingly, the described routes of administration are merely exemplary and are in no way limiting.

No matter the route of administration, the expression vector of the inventive method must reach the sensory epithelium of the inner ear. The most direct routes of administration, therefore, entail surgical procedures which allow access to the interior of the structures of the inner ear. Inoculation via cochleostomy allows administration of the expression vector directly to the regions of the inner ear associated with hearing. Cochleostomy involves drilling a hole through the cochlear wall, e.g., in the otic capsule below the stapedial artery as described in Kawamoto et al., Molecular Therapy, 4(6), 575-585 (2001), and release of a pharmaceutical composition comprising the expression vector. Administration to the endolymphatic compartment is particularly useful for administering the adenoviral vector to the areas of the inner ear responsible for hearing. Alternatively, the expression vector can be administered to the semicircular canals via canalostomy. Canalostomy provides for transgene expression in the vestibular system and the cochlea, whereas cochleostomy does not provide as efficient transduction in the vestibular space. The risk of damage to cochlear function is reduced using canalostomy in as much as direct injection into the cochlear space can result in mechanical damage to hair cells (Kawamoto et al., *supra*). Administration procedures also can be performed under fluid (e.g., artificial perilymph), which can comprise factors to alleviate side effects of treatment or the administration procedure, such as apoptosis inhibitors or anti-inflammatories.

Another direct route of administration to the inner ear is through the round window, either by injection or topical application to the round window. Administration via the round window is especially preferred for delivering an adenoviral vector to the perilymphatic space. Transgene expression in cochlear and vestibular neurons and cochlear sensory epithelia has been observed following administration of expression vectors via the round window (Staecker et al., Acta Otolaryngol, 121, 157-163 (2001)). Surprisingly, it appears possible that uptake of expression vectors, in particular non-targeted adenoviral vectors, into cells of the inner ear is not receptor-mediated. In other words, it does not appear that adenoviral infection of cells of the inner ear is mediated by CAR or integrins. To increase transduction of cells in the Organ of Corti following administration to the perilymphatic compartment, the adenoviral vector can display one or more ligands that enhance uptake of the adenoviral vector into target cells (e.g., supporting cells, cells of the stria vascularis, etc.). In this regard, the adenoviral vector can comprise one or more adenoviral coat proteins which are modified to reduce native binding (e.g., CAR-and/or integrin- binding) and comprise a non-native amino acid sequence which enhances uptake of the adenoviral vector by target cells of the inner ear.

The expression vector (e.g., adenoviral vector) is present in a pharmaceutical composition for administration to the inner ear. In certain cases, it may be appropriate to administer multiple applications and/or employ multiple routes, e.g., canalostomy and cochleostomy, to ensure sufficient exposure of supporting cells to the expression vector.

The expression vector can be present in or on a device that allows controlled or sustained release of the expression vector, such as an sponge, meshwork, mechanical reservoir or pump, or mechanical implant. For example, a biocompatible sponge or gelform soaked in a pharmaceutical composition comprising the expression vector encoding an atonal-associated factor is placed adjacent to the round window, through which the expression vector permeates to reach the cochlea (as described in Jero et al., *supra*). Mini-osmotic pumps provide sustained release of an expression vector over extended periods of time (e.g., five to seven days), allowing small volumes of composition comprising the expression vector to be administered, which can prevent mechanical damage to endogenous sensory cells. The expression vector also can be administered in the form of sustained-release formulations (see, e.g., U.S. Patent 5,378,475) comprising, for example, gelatin, chondroitin sulfate, a polyphosphoester, such as bis-2-hydroxyethyl-terephthalate (BHET), or a polylactic-glycolic acid.

While not particularly preferred, the expression vector can be administered parenterally, intramuscularly, intravenously, or intraperitoneally. Preferably, any expression vector parenterally administered to a patient for generating sensory hair cells in the ear is specifically targeted to sensory epithelial cells, such as supporting cells. Desirably, the expression vector is targeted to scarred sensory epithelium to promote generation of exogenous hair cells to replace damaged endogenous hair cells. As discussed herein, an expression vector can be modified to alter the binding specificity or recognition of an expression vector for a receptor on a potential host cell. With respect to adenovirus, such manipulations can include deletion of regions of the fiber, penton, or hexon, insertions of various native or non-native ligands into portions of the coat protein, and the like. One of ordinary skill in the art will appreciate that parenteral administration can require large doses or multiple administrations to effectively deliver the expression vector to the appropriate host cells. Pharmaceutically acceptable carriers for compositions are well-known to those of ordinary skill in the art (see Pharmaceutics and Pharmacy Practice, J.B. Lippincott Co., Philadelphia, PA, Banker and Chalmers, eds., pages 238-250 (1982), and ASHP Handbook on Injectable Drugs, Toissel, 4th ed., pages 622-630 (1986)). Although less preferred, the expression vector can also be administered *in vivo* by particle bombardment, i.e., a gene gun.

One of ordinary skill in the art also will appreciate that dosage and routes of administration can be selected to minimize loss of expression vector due to a host's immune system. For example, for contacting target cells *in vivo,* it can be advantageous to administer to a host a null expression vector (i.e., an expression vector not comprising the nucleic acid sequence encoding an atonal-associated factor prior to performing the inventive method. Prior administration of null expression vectors can serve to create an immunity in the host to the expression vector hinder the body's innate clearance mechanisms, thereby decreasing the amount of vector cleared by the immune system.

### Dosage

The dose of expression vector administered to an animal, particularly a human, in accordance with the invention should be sufficient to affect the desired response in the animal over a reasonable time frame. One skilled in the art will recognize that dosage will depend upon a variety of factors, including the age, species, location of damaged sensory epithelia, the pathology in question (if any), and condition or disease state. Dosage also depends on the atonal-associated factor, as well as the amount of sensory epithelium to be transduced. The size of the dose also will be determined by the route, timing, and frequency of administration as well as the existence, nature, and extent of any adverse side effects that might accompany the administration of a particular expression vector (e.g., surgical trauma) and the desired physiological effect. It will be appreciated by one of ordinary skill in the art that various conditions or disease states, in particular, chronic conditions or disease states, may require prolonged treatment involving multiple administrations.

Suitable doses and dosage regimens can be determined by conventional range-finding techniques known to those of ordinary skill in the art. When the expression vector is a viral vector, most preferably an adenoviral vector, about 10⁵ viral particles to about 10¹² viral particles are delivered to the patient. In other words, a pharmaceutical composition can be administered that comprises an expression vector concentration of about 10⁵ particles/ml to about 10¹³ particles/ml (including all integers within the range of about 10⁵ particles/ml to about 10¹³ particles/ml), preferably about 10¹⁰ particles/ml to about 10¹² particles/ml, and will typically involve the administration of about 0.1 µl to about 100 µl of such a pharmaceutical composition directly to the inner ear. In view of the above, the dose of one
administration preferably is at least about 1x10° particles (e.g., about 4x10⁶-4x10¹² particles), more preferably at least about 1x10⁷ particles, more preferably at least about 1x10⁸ particles (e.g., about 4x10⁸-4x10¹¹ particles), and most preferably at least about 1x10⁹ particles to at least about 1x10¹⁰ particles (e.g., about 4x10⁹-4x10¹⁰ particles)
of an adenoviral vector comprising a nucleic acid sequence encoding an atonal-associated factor. Alternatively, the dose of the pharmaceutical composition comprises no more than about 1x10¹⁴ particles, preferably no more than about 1x10¹³ particles, even more preferably no more than about 1x10¹² particles, even more preferably no more than about 1x10¹¹ particles, and most preferably no more than about 1x10¹⁰ particles (e.g., no more than about 1x10⁹ particles). In other words, a single dose of pharmaceutical composition can comprise about 1x10⁶ particle units (pu), 4x10⁶ pu, 1x10⁷ pu, 4x10⁷ pu, 1x10⁸ pu, 4x10⁸ pu, 1x10⁹ pu, 4x10⁹ pu, 1x10¹⁰ pu, 4x10¹⁰ pu, 1x10¹¹ pu, 4x10¹¹ pu, 1x10¹¹ pu, 4x10¹¹ pu, 1x10¹² pu, or 4x10¹² pu of the adenoviral vector (e.g., the replication-deficient adenoviral vector). When the expression vector is a plasmid, preferably about 0.5 ng to about 1000 µg of DNA is administered. More preferably, about 0.1 µg to about 500 µg is administered, even more preferably about 1 µg to about 100 µg of DNA is administered. Most preferably, about 50 µg of DNA is administered to the inner ear. Of course, other routes of administration may require smaller or larger doses to achieve a therapeutic effect. Any necessary variations in dosages and routes of administration can be determined by the ordinarily skilled artisan using routine techniques known in the art.

The interior space of the structures of the inner ear is limited. The volume of pharmaceutical composition administered directly into the inner ear structures should be carefully monitored, as forcing too much composition will damage the sensory epithelium. For a human patient, the volume administered is preferably about 10 ml to about 2 ml (e.g., from about 25 ml to about 1.5 ml) of composition. For example, from about 50 ml to about 1 ml (e.g., about 100 ml, 200 ml, 300 ml, 400 ml, 500 ml, 600 ml, 700 ml, 800 ml, or 900 ml) of composition can be administered. In one embodiment, the entire fluid contents of the inner ear structure, e.g., the cochlea or semi-circular canals, is replaced with pharmaceutical composition. In another embodiment, a pharmaceutical composition comprising the expression vector of the inventive method is slowly released into the inner ear structure, such that mechanical trauma is minimized.

It can be advantageous to administer two or more (i.e., multiple) doses of the expression vector comprising a nucleic acid sequence encoding an atonal-associated factor. The inventive method provides for administration of multiple doses of the nucleic acid sequence encoding an atonal-associated factor to generate hair cells in the sensory epithelium to change the sensory perception of an animal. For example, at least two doses of an expression vector comprising an exogenous nucleic acid, e.g., a nucleic acid sequence encoding an atonal-associated factor, can be administered to the same ear. Preferably, the multiple doses are administered while retaining gene expression above background levels. Also preferably, the sensory epithelium of the inner ear is contacted with two doses or more of the expression vector within about 30 days. More preferably, two or more applications are administered to the inner ear within about 90 days. However, three, four, five, six, or more doses can be administered in any time frame (e.g., 2, 7, 10, 14, 21, 28, 35, 42, 49, 56, 63, 70, 77, 85 or more days between doses) so long as gene expression occurs.

### Pharmaceutical Composition

The expression vector of the invention desirably is administered in a pharmaceutical composition, which comprises a pharmaceutically acceptable carrier and the expression vector(s). Any suitable pharmaceutically acceptable carrier can be used within the context of the invention, and such carriers are well known in the art. The choice of carrier will be determined, in part, by the particular site to which the composition is to be administered and the particular method used to administer the composition. Ideally, in the context of adenoviral vectors, the pharmaceutical composition preferably is free of replication-competent adenovirus.

Suitable formulations include aqueous and non-aqueous solutions, isotonic sterile solutions, which can contain anti-oxidants, buffers, bacteriostats, and solutes that render the formulation isotonic with the blood or fluid of the inner ear of the intended recipient, and aqueous and non-aqueous sterile suspensions that can include suspending agents, solubilizers, thickening agents, stabilizers, and preservatives. The formulation can include artificial endolymph or perilymph, which are commercially available. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets of the kind previously described. Preferably, the pharmaceutically acceptable carrier is a buffered saline solution. More preferably, the expression vector for use in the inventive method is administered in a pharmaceutical composition formulated to protect the expression vector from damage prior to administration. For example, the pharmaceutical composition can be formulated to reduce loss of the expression vector on devices used to prepare, store, or administer the expression vector, such as glassware, syringes, or needles. The pharmaceutical composition can be formulated to decrease the light sensitivity and/or temperature sensitivity of the expression vector. To this end, the pharmaceutical composition preferably comprises a pharmaceutically acceptable liquid carrier, such as, for example, those described above, and a stabilizing agent selected from the group consisting of polysorbate 80, L-arginine, polyvinylpyrrolidone, trehalose, and combinations thereof. Use of such a pharmaceutical composition will extend the shelf life of the vector, facilitate administration, and increase the efficiency of the inventive method. In this regard, a pharmaceutical composition also can be formulated to enhance transduction efficiency.
In addition, one of ordinary skill in the art will appreciate that the expression vector, e.g., viral vector, can be present in a composition with other therapeutic or biologically-active agents. For example, therapeutic factors useful in the treatment of a particular indication can be present. Factors that control inflammation, such as ibuprofen or steroids, can be part of the composition to reduce swelling and inflammation associated with *in vivo* administration of the viral vector. Immune system suppressors can be administered in combination with the inventive method to reduce any immune response to the vector itself or associated with a disorder of the inner ear. Angiogenic factors, neurotrophic factors, proliferating agents, and the like can be present in the pharmaceutical composition. Similarly, vitamins and minerals, anti-oxidants, and micronutrients can be co-administered. Antibiotics, i.e., microbicides and fungicides, can be present to reduce the risk of infection associated with gene transfer procedures and other disorders.

### Other Considerations

The inventive composition is used for administering to the inner ear an expression vector comprising nucleic acid sequence encoding an atonal-associated factor to change the sensory perception of an animal by generating hair cells in the sensory epithelium of the inner ear. The nucleic acid sequence encoding the atonal-associated factor can encode multiple (i.e., two, three, or more) atonal-associated factors, or multiple copies of the same atonal-associated factor. However, the mere generation of a hair cell does not ensure a change in sensory perception in an animal. A sufficient number of hair cells must be generated, and those sensory hair cells must be linked to a neural network capable of transmitting signals to the brain. Accordingly, while not required, it may be advantageous to provide additional factors to ensure proper reception and transmission of signals to the brain.

Several options are available for delivering multiple coding sequences to the inner ear. The nucleic acid sequence encoding the atonal-associated factor can encode additional gene products. The expression vector alternatively, or in addition, can comprise multiple expression cassettes encoding different gene products. Multiple coding sequences can be operably linked to different promoters, e.g., different promoters having dissimilar levels and patterns of activity. Alternatively, the multiple coding sequences can be operably linked to the same promoter to form a polycistronic element. The invention also contemplates administering to the inner ear a cocktail of expression vectors, wherein each expression vector encodes an atonal-associated factor or another gene product beneficial to sensory perception. The cocktail of expression vectors can further comprise different types of expression vectors, e.g., adenoviral vectors and adeno-associated viral vectors.

In one preferred embodiment, the inventive composition also contemplates delivery of a nucleic acid sequence encoding at least one neurotrophic agent. Ideally, the neurotrophic agent is a neural growth stimulator, which induces growth, development, and/or maturation of neural processes. Neurotrophic factors also can be administered to protect or maintain existing and developing neurons. For a newly generated hair cell to function properly, a neural network must be in place to transmit neural impulses to the brain. Accordingly, it is advantageous to protect existing neurons associated with the sensory epithelium of the inner ear while generating new hair cells, induce the growth and maturation of new neural processes, and/or simply direct existing neural processes to sensory hair cells. Neurotrophic factors are divided into three subclasses: neuropoietic cytokines; neurotrophins; and the fibroblast growth factors. Ciliary neurotrophic factor (CNTF) is exemplary of neuropoietic cytokines. CNTF promotes the survival of ciliary ganglionic neurons and supports certain neurons that are NGF-responsive. Neurotrophins include, for example, brain-derived neurotrophic factor (BDNF) and nerve growth factor (NGF), which stimulates neurite outgrowth. Other neurotrophic factors include, for example, transforming growth factors, glial cell-line derived neurotrophic factor (GDNF), neurotrophin 3, neurotrophin 4/5, and interleukin 1-β. Neuronotrophic factors enhance neuronal survival and also are suitable for use in the inventive method. It has been postulated that neuronotrophic factors can actually reverse degradation of neurons. Such factors, conceivably, are useful in treating the degeneration of neurons associated with age, infection, or trauma. A preferred neuronotrophic factor is pigment epithelium derived factor (PEDF). PEDF is further described in Chader, Cell Different., 20, 209-216 (1987), Pignolo et al., J. Biol. Chem., 268(12), 8949-8957 (1998), U.S. Patent 5,840,686, and International Patent Applications WO 93/24529, WO 99/04806, and WO 01/58494.

Proliferating agents induce cellular proliferation, preferably proliferation of supporting cells in the inner ear. Multiplying the number of hair cell progenitors maximizes the biological effect of the atonal-associated factor. Supporting cell proliferation is induced by mitogenic growth factors, such as fibroblast growth factors (FGF, in particular FGF-2), vascular endothelial growth factors (VEGF), epidermal growth factor (EGF), E2F, cell cycle up-regulators, and the like. A nucleic acid sequence encoding a proliferating agent can be administered in conjunction with the nucleic acid sequence encoding an atonal-associated factor in the inventive method. If desired, the nucleic acid sequence encoding a proliferating agent can be engineered to exert its biological effect only on the cell type to be replicated. For supporting cells, the nucleic acid can comprise a regulatory sequence that is preferentially activated in supporting cells, e.g., a promoter that is active only in the presence of *hes* transcription factors. The resulting proliferating agent also can be engineered to prevent secretion into the cellular milieu. Alternatively, a substance can be administered to the inner ear to promote cell proliferation or enhance uptake of the expression vector.

In addition to the above, one or more other transgenes can be carried by the same nucleic acid sequence that encodes the atonal-associated factor or can be a separate nucleic acid sequence present on the same expression vector or part of a different expression vector. By "transgene" is meant any nucleic acid that can be expressed in a cell. Desirably, the expression of the transgene is beneficial, e.g., prophylactically or therapeutically beneficial, to the inner ear. If the transgene confers a prophylactic or therapeutic benefit to a cell, the transgene can exert its effect at the level of RNA or protein. For example, the transgene can encode a peptide other than an atonal-associated factor that can be employed in the treatment or study of a disorder, e.g., a sensory disorder stemming from abnormalities in the inner ear. Alternatively, the transgene can encode an antisense molecule, a ribozyme, a protein that affects splicing or 3' processing (e.g., polyadenylation), or a protein that affects the level of expression of another gene within the cell (i.e., where gene expression is broadly considered to include all steps from initiation of transcription through production of a process protein), such as by mediating an altered rate of mRNA accumulation or transport or an alteration in post-transcriptional regulation. The transgene can encode a chimeric peptide for combination treatment of an inner ear-related disorder. The transgene can encode a factor that acts upon a different target molecule than the atonal-associated factor, or initiates a signal transduction cascade not affected by the atonal-associated factor. The transgene can encode a marker protein, such as green fluorescent protein or luciferase. Such marker proteins are useful in vector construction and determining vector migration. Alternatively, the transgene can encode a selection factor, which also is useful in vector construction protocols and can be employed to select against non-transduced cells.

The composition of the invention can be part of a treatment regimen involving other therapeutic modalities. It is appropriate, therefore, if the inventive composition is employed to prophylactically or therapeutically treat a sensory disorder, namely a hearing disorder or a balance disorder, that has been treated, is being treated, or will be treated with any of a number of other therapies, such as drug therapy or surgery. The inventive composition also can be used in conjunction with the implantation of hearing devices, such as cochlear implants. The inventive composition also is particularly suited for procedures involving stem cells to regenerate populations of cells within the inner ear. In this respect, the inventive composition can be used *ex vivo* to transduce stem cells, which are then implanted within the inner ear.

The expression vector is preferably administered as soon as possible after it has been determined that an animal, such as a mammal, specifically a human, is at risk for degeneration of sensory hair cells (prophylactic treatment) or has demonstrated reduced numbers or damage of sensory hair cells (therapeutic treatment). Treatment will depend, in part, upon the particular nucleic acid sequence used, the particular atonal-associated factor expressed from the nucleic acid sequence, the expression vector, the route of administration, and the cause and extent, if any, of hair cell loss or damage realized.

An expression vector comprising a nucleic acid sequence encoding an atonal-associated factor can be introduced *ex vivo* into cells previously removed from a given animal, in particular a human. Such transduced autologous or homologous host cells can be progenitor cells that are reintroduced into the inner ear of the animal or human to express the atonal-associated factor and differentiate into mature hair cells *in vivo.* One of ordinary skill in the art will understand that such cells need not be isolated from the patient, but can instead be isolated from another individual and implanted into the patient.

The inventive composition also can be co-administered with other pharmaceutically active compounds. By "co-administration" is meant administration before, concurrently with, e.g., in combination with the expression vector in the same formulation or in separate formulations, or after administration of the expression vector as described above. For example, factors that control inflammation, such as ibuprofen or steroids, can be co-administered to reduce swelling and inflammation associated with administration of the expression vector. Immunosuppressive agents can be co-administered to reduce inappropriate immune responses related to an inner ear disorder or the practice of the inventive method. Similarly, vitamins and minerals, anti-oxidants, and micronutrients can be co-administered. Antibiotics, i.e., microbicides and fungicides, can be co-administered to reduce the risk of infection associated with surgical procedures.

### EXAMPLES

The following examples further illustrate the invention but, of course, should not be construed as in any way limiting its scope.

### EXAMPLE 1

This example demonstrates that adenoviral vectors transduce cells of the mammalian inner ear and drive expression of a transgene using several different promoters.

Adult utricles or P3 rat cochleae were cultured on Millicel membranes in Dulbecco Modified Eagle Medium supplemented with N1 and glucose. Cultures were maintained under standard conditions, and medium was changed every three days. Adenoviral vector backbones lacking all or part of the E1 and E3 regions of the adenoviral genome (AdL.10) or lacking all or part of the E1, E3, and E4 regions of the adenoviral genome and comprising a spacer located in place of the E4 region (AdL.11D) were constructed and produced as previously described (see, e.g., U.S. Patents 5,851,806 and 5,994,106). Six adenoviral vector constructs were prepared using either the AdL.10 or AdL.11D vector backbones. Each adenoviral vector construct comprised the luciferase reporter gene operably linked to a one of six promoters, human cytomegalovirus immediate early promoter (AdhCMV.L), murine cytomegalovirus immediate early promoter (AdmCMV.L), ubiquitin promoter (AdUb.L), chicken b-actin promoter (AdBA.L), rous sarcoma virus promoter (AdRSV.L), or the p5 promoter from AAV (Adp5.L).

Luciferase expression was determined by extracting the entire utricle or cochleae explant in reporter lysis buffer, and the amount of total protein was determined by Bio-Rad protein assay. The amount of luciferase activity was determined by luminescence and expressed as relative light units per mg of total protein.

Organ of Corti (cochlear) explants from P3 mice were transduced with 1x10⁹ particle units (pu) of AdhCMV.L.11D, AdmCMV.L.11D, AdUb.L.11D, AdBA.L.11D, AdRSV.L.11D, or Adp5.L.11D. At four days post-administration, the explant cultures were evaluated for the transgene expression. The highest observed level of transgene expression was mediated by AdhCMV.L.1 ID and AdBA.L.11D. In addition, the level of transgene expression was followed over 28 days post-administration of AdhCMV.L.1 ID, AdmCMV.L.11D, and AdBA.L.11D. For each adenoviral vector construct, transgene expression wanes between Day 1 and Day 14 post-administration but remains steady thereafter from Day 14 through Day 28. However, transgene expression mediated by AdhCMV.L.11D, AdUb.L.11D, and AdBA.L.11D in adult C57B6 mouse utricle explants was either constant (AdhCMV.L.11D) or increased (AdUb.L.11D, AdBA.L.11D) between Day 1 and Day 14 post-administration. Adenoviral vector-mediated transgene expression was observed in utricle explant culture remained stable for approximately 5 weeks post-administration, and declined to undetectable levels by Week 7 post-administration.

This example demonstrates that replication-deficient adenoviral vectors transduce cells of the cochlea and vestibular system *in vitro.* Adenoviral vector-mediated transgene expression can be observed using a variety of promoters, and was observed for at least 5 weeks post-administration

### EXAMPLE 2

This example demonstrates that adenoviral-mediated production of Math 1 directs production of new hair cells in a mammal.

An E1/E3/E4-deficient adenoviral vector comprising Math1 cDNA operably linked to the cytomegalovirus immediate early (CMV) promoter and inserted into the E1 region of the adenoviral genome (AdMath1.11D) was constructed using methods set forth in Brough et al., J. Virol., 71, 9206-9213 (1997) and Mori et al., J. Cell. Physiol., 188, 253-263 (2001). AdMath1.11D was surgically injected into the endolymphatic fluid of the third cochlear turn of the scala media in the mature guinea pig cochlea at a rate of 5 ml in 5 minutes (5x10¹¹ particles/ml of composition). As the volume of composition administered was greater than the volume of endolymph present in the scala media, injection of the adenoviral vector composition resulted in mechanical damage to hair cells lining the scala media. Control ears were injected with either endolymph alone or AdMath1.11D vector constructs lacking Math1 cDNA (AdNull).

The surface of the organ of Corti was analyzed at 30 or 60 days post-administration of the AdMath1.11D using scanning electron microscopy. Sensory hair cell generation was detected in all treated guinea pigs. Hair cells were observed in regions where hair cells are typically absent, adjacent to the organ of Corti sensory epithelium. Hair cells also were observed in the inner sulcus, interdental cell regions, and Hensen cell region located lateral to the organ of Corti. Based on their location, the sensory hair cells were newly generated as a result of AdMath1.11D treatment. Injection with endolymph or AdNull did not promote hair cell generation in these regions. Animals sacrificed at 60 days post-administration of AdMath1.11D had more newly generated sensory hair cells than animals sacrificed at 30 days-post administration.

The surface morphology of the sensory hair cells appeared nearly normal. The degree of differentiation of sensory hair cells in the inner sulcus, interdental region, and Hensen cell region was determined by immunocytochemistry using anti-myosin VIIa antibodies (Hasson et al. Proc. Natl. Acad. Sci., 92, 9815-9819 (1995)). Myosin VIIa positive cells were identified medial to the OC, in the area of the inner sulcus and interdental region, and lateral to the organ of Corti in the Hensen cell region. Expression of myosin VIIa in these cells further confirms that the cells were differentiated sensory hair cells.

To determine if neural processes grow toward sensory hair cells generated by Math1 expression, control cochlea and cochleae treated with AdMath1.11D were double-stained with anti-myosin VIIa and anti-neurofilament antibodies. In control tissues, neurofilament staining was abundant in the area of the OC, revealing radial and longitudinal neural fibers. Neurofilament staining was absent in the inner sulcus and interdental cell regions of the control cochleae inoculated with endolymph along or AdNull. In contrast, neurofilament-stained processes extending from the area of the OC in the direction of sensory hair cells in the interdental cell region was observed in tissues treated with AdMath1.11D. Staining demonstrated that axons are attracted to sensory hair cells in the cochlea.

This example demonstrates that non-sensory cells in the mature mammalian cochlea can be induced to differentiate into sensory hair cells by adenoviral-mediated expression of Math1, and that neurons in mature animals are attracted to and extend in the direction of sensory hair cells.

### EXAMPLE 3

This example demonstrates that the inventive method can affect the sensory perception in an animal by restoring, at least in part, balance.

AdMath1.11D, an adenoviral vector deficient in all of the replication-essential gene functions of the E1 and E4 regions of the adenoviral genome, further lacking a majority of the E3 region of the adenoviral genome, and comprising Math1 cDNA operably linked to the CMV promoter, were constructed as described in Example 2.

Neomycin was administered into the inner ear of mice, thereby damaging sensory hair cells in the vestibular system resulting in loss of balance awareness. A subset of mice were administered 1x10⁸-2x10⁸ AdMath1.11D in 1 ml of composition via delivery through the round window and into the perilymph of the semicircular canals. Evaluation of balance of body position awareness in mice was accomplished using a "swim test." When placed in a tank of water, untreated control animals (i.e., normal mice) required approximately 8.5 seconds to right themselves and begin swimming purposely. Neomycin treatment damages sensory hair cells in the vestibular system. Mice treated with neomycin required 22.2 seconds right themselves and swim purposely due to the dysfunction of vestibular control. In contrast, mice treated with neomycin and AdMath1.11D required only 12 seconds to right themselves and swim purposely, suggesting functional recovery of vestibular function.

This example confirms that the inventive composition can restore vestibular function following damage to sensory hair cells, thereby changing the sensory perception of an animal.

### EXAMPLE 4

This example illustrates a method of delivering an adenoviral vector comprising a nucleic acid sequence encoding Math1 to the inner ear.

Normal adult guinea pigs were deafened by subcutaneous administration of kanamycin (500 mg/kg). Ethacrynic acid (50 mg/kg) was administered two hours later via jugular vein infusion. Treatment with kanamycin and ethacrinic acid resulted in bilateral hair loss in the high and mid-frequency regions of the cochlea, leaving only supporting cells in the epithelium. Outer hair cells are destroyed throughout the cochlea, and inner hair cells are destroyed in the first three cochlear turns. Four days following deafening, Ad.Math1.11D, described above, was injected into the second turn of the scala media (i.e., the endolymphatic compartment). Contralateral ears were not treated. To examine hearing restoration, ABRs were performed at several pure tone signals.

Newly differentiated, Math1-positive hair cells were detected in the first, second, and third cochlear turns at two-months post-administration, and directly correlated with the density of cells transduced by Ad.Math1.11D. One month following administration of Ad.Math1.11D, average ABR thresholds improved in treated ears as compared to ABR thresholds of untreated, contralateral ears. ABR thresholds were further improved at two months post-vector administration in treated ears.

This example demonstrates that adenovira1-mediated delivery of Math1 to mature and damaged epithelium of the inner ear promotes differentiation of non-sensory cochlear cells to new hair cells. The generation of new hair cells results in restoration of function of the inner ear.

The use of the terms "a" and "an" and "the" and similar references in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A pharmaceutical composition in the form of a solution for administration to the inner ear, designed for changing the sensory perception of an animal, including a human, comprising a serotype 5 adenoviral vector comprising a nucleic acid sequence encoding a human atonal homolog 1 (Hath1) protein operably linked to a promoter that functions in supporting cells of the inner ear,
wherein the nucleic acid sequence is expressed to produce the Hath1 protein resulting in generation of sensory hair cells that allow perception of stimuli in the inner ear, and
wherein the adenoviral vector comprises (i) a deletion of the E1 region of the serotype 5 adenoviral genome, (ii) a deletion of the E3 region of the serotype 5 adenoviral genome, and (iii) a deletion of the E4 region of the serotype 5 adenoviral genome.

2. The pharmaceutical composition of claim 1, wherein the concentration of the adenoviral vector is from about 10⁵ particles/mL to about 10¹³ particles/mL.

3. The pharmaceutical composition of claim 1 or 2, wherein the concentration of the adenoviral vector is from about 10¹⁰ particles/mL to about 10¹² particles/mL.

4. The pharmaceutical composition of any one of claims 1-3, wherein the pharmaceutical composition comprises at least about 1x10⁶ particles of the adenoviral vector.

5. The pharmaceutical composition of any one of claims 1-4, wherein the pharmaceutical composition comprises about 4x10⁶ to 4x10¹² particles of the adenoviral vector.

6. The pharmaceutical composition of any one of claims 1-3, wherein the volume of the pharmaceutical composition is from about 0.1 µL to about 100 µL.

7. The pharmaceutical composition of any one of claims 1-6, wherein the adenoviral vector comprises a spacer inserted into the deleted E4 region.

## Patentansprüche

1. Pharmazeutische Zusammensetzung in Form einer Lösung zur Verabreichung in das Innenohr, bestimmt für die Änderung der sensorischen Wahrnehmung eines Tieres einschließlich eines Menschen, umfassend einen adenoviralen Vektor vom Serotyp 5 mit einer Nukleinsäuresequenz, die für das humane Atonal-Homolog-1-Protein (Hath1) kodiert, funktional gekoppelt an einen Promotor, der in Stützzellen des Innenohrs wirkt,
wobei die Nukleinsäuresequenz exprimiert wird, um das Hath1-Protein zu produzieren, resultierend in der Bildung von Sinneshaarzellen, welche die Wahrnehmung von Stimuli im Innenohr erlauben, und
wobei der adenovirale Vektor (i) eine Deletion der E1-Region des adenoviralen Genoms vom Serotyp 5, (ii) eine Deletion der E3-Region des adenoviralen Genoms vom Serotyp 5 und (iii) eine Deletion der E4-Region des adenoviralen Genoms vom Serotyp 5 umfasst.

2. Pharmazeutische Zusammensetzung nach Anspruch 1, wobei die Konzentration des adenoviralen Vektors von ca. 10⁵ Partikel/ml bis 10¹³ Partikel/ml beträgt.

3. Pharmazeutische Zusammensetzung nach Anspruch 1 oder 2, wobei die Konzentration des adenoviralen Vektors von ca. 10¹⁰ Partikel/ml bis ca. 10¹² Partikel/ml beträgt.

4. Pharmazeutische Zusammensetzung nach einem der Ansprüche 1 bis 3, wobei die pharmazeutische Zusammensetzung mindestens 1x10⁶ Partikel des adenoviralen Vektors umfasst.

5. Pharmazeutische Zusammensetzung nach einem der Ansprüche 1 bis 4, wobei die pharmazeutische Zusammensetzung ca. 4x10⁶ bis 4x10¹² Partikel des adenoviralen Vektors umfasst.

6. Pharmazeutische Zusammensetzung nach einem der Ansprüche 1 bis 3, wobei das Volumen der pharmazeutischen Zusammensetzung von ca. 0,1 µl bis ca. 100 µl beträgt.

7. Pharmazeutische Zusammensetzung nach einem der Ansprüche 1 bis 6, wobei der adenovirale Vektor einen in die deletierte E4-Region insertierten Spacer umfasst.

## Revendications

1. Composition pharmaceutique sous la forme d'une solution pour une administration dans l'oreille interne, conçue pour modifier la perception sensorielle d'un animal, y compris d'un être humain, comprenant un vecteur adénoviral de sérotype 5 comprenant une séquence d'acide nucléique codant pour une protéine de type homologue 1 atonal humain (Hath 1) liée de manière fonctionnelle à un promoteur qui fonctionne dans des cellules de support de l'oreille interne,
où la séquence d'acide nucléique est exprimée pour produire la protéine Hath 1 entraînant la génération de cellules ciliées sensorielles qui permettent la perception de stimuli dans l'oreille interne, et
où le vecteur adénoviral comprend (i) une délétion de la région E1 du génome adénoviral de sérotype 5, (ii) une délétion de la région E3 du génome adénoviral de sérotype 5, et (iii) une délétion de la région E4 du génome adénoviral de sérotype 5.

2. Composition pharmaceutique selon la revendication 1, dans laquelle la concentration du vecteur adénoviral est d'environ 10⁵ à environ 10¹³ particules/mL.

3. Composition pharmaceutique selon la revendication 1 ou 2, dans laquelle la concentration du vecteur adénoviral est d'environ 10¹⁰ à environ 10¹² particules/mL.

4. Composition pharmaceutique selon l'une quelconque des revendications 1 à 3, dans laquelle la composition pharmaceutique comprend au moins environ 1 x 10⁶ particules du vecteur adénoviral.

5. Composition pharmaceutique selon l'une quelconque des revendications 1 à 4, dans laquelle la composition pharmaceutique comprend d'environ 4 x 10⁶ à 4 x 10¹² particules du vecteur adénoviral.

6. Composition pharmaceutique selon l'une quelconque des revendications 1 à 3, dans laquelle le volume de la composition pharmaceutique est d'environ 0,1 à environ 100 µL.

7. Composition pharmaceutique selon l'une quelconque des revendications 1 à 6, dans laquelle le vecteur adénoviral comprend un espaceur inséré dans la région E4 délétée.


### SEQUENCE LISTING

<110> GENVEC, INC.
<120> METHODS AND MATERIALS FOR TREATING DISORDERS OF THE EAR
<130> A 7 914 p
<150> US 10/373,249
   <151> 2003-02-24
<160> 7
<170> PatentIn version 3.3
<210> 1
   <211> 351
   <212> PRT
   <213> Mus musculus
<400> 1
   Met Ser Arg Leu Leu His Ala Glu Glu Trp Ala Glu Val Lys Glu Leu
   1               5                   10                  15
   Gly Asp His His Arg His Pro Gln Pro His His Val Pro Pro Leu Thr
               20                  25                  30
   Pro Gln Pro Pro Ala Thr Leu Gln Ala Arg Asp Leu Pro Val Tyr Pro
           35                  40                  45
   Ala Glu Leu Ser Leu Leu Asp Ser Thr Asp Pro Arg Ala Trp Leu Thr
       50                  55                  60
   Pro Thr Leu Gln Gly Leu Cys Thr Ala Arg Ala Ala Gln Tyr Leu Leu
   65                  70                  75                  80
   His Ser Pro Glu Leu Gly Ala Ser Glu Ala Ala Ala Pro Arg Asp Glu
                   85                  90                  95
   Ala Asp Ser Gln Gly Glu Leu Val Arg Arg Ser Gly Cys Gly Gly Leu
               100                 105                 110
   Ser Lys Ser Pro Gly Pro Val Lys Val Arg Glu Gln Leu Cys Lys Leu
           115                 120                 125
   Lys Gly Gly Val Val Val Asp Glu Leu Gly Cys Ser Arg Gln Arg Ala
       130                 135                 140
   Pro Ser Ser Lys Gln Val Asn Gly Val Gln Lys Gln Arg Arg Leu Ala
   145                 150                 155                 160
   Ala Asn Ala Arg Glu Arg Arg Arg Met His Gly Leu Asn His Ala Phe
                   165                 170                 175
   Asp Gln Leu Arg Asn Val Ile Pro Ser Phe Asn Asn Asp Lys Lys Leu
               180                 185                 190
   Ser Lys Tyr Glu Thr Leu Gln Met Ala Gln Ile Tyr Ile Asn Ala Leu
           195                 200                 205
   Ser Glu Leu Leu Gln Thr Pro Asn Val Gly Glu Gln Pro Pro Pro Pro
       210                 215                 220
   Thr Ala Ser Cys Lys Asn Asp His His His Leu Arg Thr Ala Ser Ser
   225                 230                 235                 240
   Tyr Glu Gly Gly Ala Gly Ala Ser Ala Val Ala Gly Ala Gln Pro Ala
                   245                 250                 255
   Pro Gly Gly Gly Pro Arg Pro Thr Pro Pro Gly Pro Cys Arg Thr Arg
               260                 265                 270
   Phe Ser Gly Pro Ala Ser Ser Gly Gly Tyr Ser Val Gln Leu Asp Ala
           275                 280                 285
   Leu His Phe Pro Ala Phe Glu Asp Arg Ala Leu Thr Ala Met Met Ala
       290                 295                 300
   Gln Lys Asp Leu Ser Pro Ser Leu Pro Gly Gly Ile Leu Gln Pro Val
   305                 310                 315                 320
   Gln Glu Asp Asn Ser Lys Thr Ser Pro Arg Ser His Arg Ser Asp Gly
                   325                 330                 335
   Glu Phe Ser Pro His Ser His Tyr Ser Asp Ser Asp Glu Ala Ser
               340                 345                 350
<210> 2
   <211> 351
   <212> PRT
   <213> Mus musculus
<400> 2
   Met Ser Arg Leu Leu His Ala Glu Glu Trp Ala Glu Val Lys Glu Leu
   1               5                   10                  15
   Gly Asp His His Arg His Pro Gln Pro His His Val Pro Pro Leu Thr
               20                  25                  30
   Pro Gln Pro Pro Ala Thr Leu Gln Ala Arg Asp Leu Pro Val Tyr Pro
           35                  40                  45
   Ala Glu Leu Ser Leu Leu Asp Ser Thr Asp Pro Arg Ala Trp Leu Thr
       50                  55                  60
   Pro Thr Leu Gln Gly Leu Cys Thr Ala Arg Ala Ala Gln Tyr Leu Leu
   65                  70                  75                  80
   His Ser Pro Glu Leu Gly Ala Ser Glu Ala Ala Ala Pro Arg Asp Glu
                   85                  90                  95
   Ala Asp Ser Gln Gly Glu Leu Val Arg Arg Ser Gly Cys Gly Gly Leu
               100                 105                 110
   Ser Lys Ser Pro Gly Pro Val Lys Val Arg Glu Gln Leu Cys Lys Leu
           115                 120                 125
   Lys Gly Gly Val Val Val Asp Glu Leu Gly Cys Ser Arg Gln Arg Ala
       130                 135                 140
   Pro Ser Ser Lys Gln Val Asn Gly Val Gln Lys Gln Arg Arg Leu Ala
   145                 150                 155                 160
   Ala Asn Ala Arg Glu Arg Arg Arg Met His Gly Leu Asn His Ala Phe
                   165                 170                 175
   Asp Gln Leu Arg Asn Val Ile Pro Ser Phe Asn Asn Asp Lys Lys Leu
               180                 185                 190
   Ser Lys Tyr Glu Thr Leu Gln Met Ala Gln Ile Tyr Ile Asn Ala Leu
           195                 200                 205
   Ser Glu Leu Leu Gln Thr Pro Asn Val Gly Glu Gln Pro Pro Pro Pro
       210                 215                 220
   Thr Ala Ser Cys Lys Asn Asp His His His Leu Arg Thr Ala Ser Ser
   225                 230                 235                 240
   Tyr Glu Gly Gly Ala Gly Ala Ser Ala Val Ala Gly Ala Gln Pro Ala
                   245                 250                 255
   Pro Gly Gly Gly Pro Arg Pro Thr Pro Pro Gly Pro Cys Arg Thr Arg
               260                 265                 270
   Phe Ser Gly Pro Ala Ser Ser Gly Gly Tyr Ser Val Gln Leu Asp Ala
           275                 280                 285
   Leu His Phe Pro Ala Phe Glu Asp Arg Ala Leu Thr Ala Met Met Ala
       290                 295                 300
   Gln Lys Asp Leu Ser Pro Ser Leu Pro Gly Gly Ile Leu Gln Pro Val
   305                 310                 315                 320
   Gln Glu Asp Asn Ser Lys Thr Ser Pro Arg Ser His Arg Ser Asp Gly
                   325                 330                 335
   Glu Phe Ser Pro His Ser His Tyr Ser Asp Ser Asp Glu Ala Ser
               340                 345                 350
<210> 3
   <211> 354
   <212> PRT
   <213> Homo sapiens
<400> 3
   Met Ser Arg Leu Leu His Ala Glu Glu Trp Ala Glu Val Lys Glu Leu
   1               5                   10                  15
   Gly Asp His His Arg Gln Pro Gln Pro His His Leu Pro Gln Pro Pro
               20                  25                  30
   Pro Pro Pro Gln Pro Pro Ala Thr Leu Gln Ala Arg Glu His Pro Val
           35                  40                  45
   Tyr Pro Pro Glu Leu Ser Leu Leu Asp Ser Thr Asp Pro Arg Ala Trp
       50                  55                  60
   Leu Ala Pro Thr Leu Gln Gly Ile Cys Thr Ala Arg Ala Ala Gln Tyr
   65                  70                  75                  80
   Leu Leu His Ser Pro Glu Leu Gly Ala Ser Glu Ala Ala Ala Pro Arg
                   85                  90                  95
   Asp Glu Val Asp Gly Arg Gly Glu Leu Val Arg Arg Ser Ser Gly Gly
               100                 105                 110
   Ala Ser Ser Ser Lys Ser Pro Gly Pro Val Lys Val Arg Glu Gln Leu
           115                 120                 125
   Cys Lys Leu Lys Gly Gly Val Val Val Asp Glu Leu Gly Cys Ser Arg
       130                 135                 140
   Gln Arg Ala Pro Ser Ser Lys Gln Val Asn Gly Val Gln Lys Gln Arg
   145                 150                 155                 160
   Arg Leu Ala Ala Asn Ala Arg Glu Arg Arg Arg Met His Gly Leu Asn
                   165                 170                 175
   His Ala Phe Asp Gln Leu Arg Asn Val Ile Pro Ser Phe Asn Asn Asp
               180                 185                 190
   Lys Lys Leu Ser Lys Tyr Glu Thr Leu Gln Met Ala Gln Ile Tyr Ile
           195                 200                 205
   Asn Ala Leu Ser Glu Leu Leu Gln Thr Pro Ser Gly Gly Glu Gln Pro
       210                 215                 220
   Pro Pro Pro Pro Ala Ser Cys Lys Ser Asp His His His Leu Arg Thr
   225                 230                 235                 240
   Ala Ala Ser Tyr Glu Gly Gly Ala Gly Asn Ala Thr Ala Ala Gly Ala
                   245                 250                 255
   Gln Gln Ala Ser Gly Gly Ser Gln Arg Pro Thr Pro Pro Gly Ser Cys
               260                 265                 270
   Arg Thr Arg Phe Ser Ala Pro Ala Ser Ala Gly Gly Tyr Ser Val Gln
           275                 280                 285
   Leu Asp Ala Leu His Phe Ser Thr Phe Glu Asp Ser Ala Leu Thr Ala
       290                 295                 300
   Met Met Ala Gln Lys Asn Leu Ser Pro Ser Leu Pro Gly Ser Ile Leu
   305                 310                 315                 320
   Gln Pro Val Gln Glu Glu Asn Ser Lys Thr Ser Pro Arg Ser His Arg
                   325                 330                 335
   Ser Asp Gly Glu Phe Ser Pro His Ser His Tyr Ser Asp Ser Asp Glu
               340                 345                 350
   Ala Ser
<210> 4
   <211> 354
   <212> PRT
   <213> Homo sapiens
<400> 4
   Met Ser Arg Leu Leu His Ala Glu Glu Trp Ala Glu Val Lys Glu Leu
   1               5                   10                  15
   Gly Asp His His Arg Gln Pro Gln Pro His His Leu Pro Gln Pro Pro
               20                  25                  30
   Pro Pro Pro Gln Pro Pro Ala Thr Leu Gln Ala Arg Glu His Pro Val
           35                  40                  45
   Tyr Pro Pro Glu Leu Ser Leu Leu Asp Ser Thr Asp Pro Arg Ala Trp
       50                  55                  60
   Leu Ala Pro Thr Leu Gln Gly Ile Cys Thr Ala Arg Ala Ala Gln Tyr
   65                  70                  75                  80
   Leu Leu His Ser Pro Glu Leu Gly Ala Ser Glu Ala Ala Ala Pro Arg
                   85                  90                  95
   Asp Glu Val Asp Gly Arg Gly Glu Leu Val Arg Arg Ser Ser Gly Gly
               100                 105                 110
   Ala Ser Ser Ser Lys Ser Pro Gly Pro Val Lys Val Arg Glu Gln Leu
           115                 120                 125
   Cys Lys Leu Lys Gly Gly Val Val Val Asp Glu Leu Gly Cys Ser Arg
       130                 135                 140
   Gln Arg Ala Pro Ser Ser Lys Gln Val Asn Gly Val Gln Lys Gln Arg
   145                 150                 155                 160
   Arg Leu Ala Ala Asn Ala Arg Glu Arg Arg Arg Met His Gly Leu Asn
                   165                 170                 175
   His Ala Phe Asp Gln Leu Arg Asn Val Ile Pro Ser Phe Asn Asn Asp
               180                 185                 190
   Lys Lys Leu Ser Lys Tyr Glu Thr Leu Gln Met Ala Gln Ile Tyr Ile
           195                 200                 205
   Asn Ala Leu Ser Glu Leu Leu Gln Thr Pro Ser Gly Gly Glu Gln Pro
       210                 215                 220
   Pro Pro Pro Pro Ala Ser Cys Lys Ser Asp His His His Leu Arg Thr
   225                 230                 235                 240
   Ala Ala Ser Tyr Glu Gly Gly Ala Gly Asn Ala Thr Ala Ala Gly Ala
                   245                 250                 255
   Gln Gln Ala Ser Gly Gly Ser Gln Arg Pro Thr Pro Pro Gly Ser Cys
               260                 265                 270
   Arg Thr Arg Phe Ser Ala Pro Ala Ser Ala Gly Gly Tyr Ser Val Gln
           275                 280                 285
   Leu Asp Ala Leu His Phe Ser Thr Phe Glu Asp Ser Ala Leu Thr Ala
       290                 295                 300
   Met Met Ala Gln Lys Asn Leu Ser Pro Ser Leu Pro Gly Ser Ile Leu
   305                 310                 315                 320
   Gln Pro Val Gln Glu Glu Asn Ser Lys Thr Ser Pro Arg Ser His Arg
                   325                 330                 335
   Ser Asp Gly Glu Phe Ser Pro His Ser His Tyr Ser Asp Ser Asp Glu
               340                 345                 350
   Ala Ser
<210> 5
   <211> 21
   <212> PRT
   <213> Drosophila
<400> 5
   Ala Ala Asn Ala Arg Glu Arg Arg Arg Met His Gly Leu Asn His Ala
   1               5                   10                  15
   Phe Asp Gln Leu Arg
               20
<210> 6
   <211> 1393
   <212> DNA
   <213> Mus musculus
<400> 6
   aagcttcgtt gcacgcgacc tggtgtgcga tctccgagtg agagggggag ggtcagagga      60
   ggaaggaaaa aaaatcagac cttgcagaag agactaggaa ggtttttgtt gttgttgttc     120
   ggggcttatc cccttcgttg aactgggttg ccagcacctc ctctaacacg gcacctccga     180
   gccattgcag tgcgatgtcc cgcctgctgc atgcagaaga gtgggctgag gtaaaagagt     240
   tgggggacca ccatcgccat ccccagccgc accacgtccc gccgctgacg ccacagccac 300
   ctgctaccct gcaggcgaga gaccttcccg tctacccggc agaactgtcc ctcctggata 360
   gcaccgaccc acgcgcctgg ctgactccca ctttgcaggg cctctgcacg gcacgcgccg 420
   cccagtatct gctgcattct cccgagctgg gtgcctccga ggccgcggcg ccccgggacg 480
   aggctgacag ccagggtgag ctggtaagga gaagcggctg tggcggcctc agcaagagcc 540
   ccgggcccgt caaagtacgg gaacagctgt gcaagctgaa gggtggggtt gtagtggacg 600
   agcttggctg cagccgccag cgagcccctt ccagcaaaca ggtgaatggg gtacagaagc 660
   aaaggaggct ggcagcaaac gcaagggaac ggcgcaggat gcacgggctg aaccacgcct 720
   tcgaccagct gcgcaacgtt atcccgtcct tcaacaacga caagaagctg tccaaatatg 780
   agaccctaca gatggcccag atctacatca acgctctgtc ggagttgctg cagactccca 840
   atgtcggaga gcaaccgccg ccgcccacag cttcctgcaa aaatgaccac catcaccttc 900
   gcaccgcctc ctcctatgaa ggaggtgcgg gcgcctctgc ggtagctggg gctcagccag 960
   ccccgggagg gggcccgaga cctaccccgc ccgggccttg ccggactcgc ttctcaggcc 1020
   cagcttcctc tgggggttac tcggtgcagc tggacgcttt gcacttccca gccttcgagg 1080
   acagggccct aacagcgatg atggcacaga aggacctgtc gccttcgctg cccgggggca 1140
   tcctgcagcc tgtacaggag gacaacagca aaacatctcc cagatcccac agaagtgacg 1200
   gagagttttc cccccactct cattacagtg actctgatga ggccagttag gaaggcaaca 1260
   gctccctgaa aactgagaca accaaatgcc cttcctagcg cgcgggaagc cccgtgacaa 1320
   atatccctgc accctttaat ttttggtctg tggtgatcgt tgttagcaac gacttgactt 1380
   cggacggctg cag 1393
<210> 7
   <211> 1572
   <212> DNA
   <213> Homo sapiens
<220>
   <221> misc_feature
   <222> (1497)..(1497)
   <223> "n" may be any nucleotide
<220>
   <221> misc_feature
   <222> (1504)..(1504)
   <223> "n" may be any nucleotide
<220>
   <221> misc_feature
   <222> (1526)..(1526)
   <223> "n" may be any nucleotide
<220>
   <221> misc_feature
   <222> (1564)..(1564)
   <223> "n" may be any nucleotide
<400> 7
   gtcctctgca cacaagaact tttctcgggg tgtaaaaact ctttgattgg ctgctcgcac      60
   gcgcctgccc gcgccctcca ttggctgaga agacacgcga ccggcgcgag gagggggttg     120
   ggagaggagc ggggggagac tgagtggcgc gtgccgcttt ttaaaggggc gcagcgcctt      180
   cagcaaccgg agaagcatag ttgcacgcga cctggtgtgt gatctccgag tgggtggggg     240
   agggtcgagg agggaaaaaa aaataagacg ttgcagaaga gacccggaaa gggccttttt     300
   tttggttgag ctggtgtccc agtgctgcct ccgatcctga gcgtccgagc ctttgcagtg     360
   caatgtcccg cctgctgcat gcagaagagt gggctgaagt gaaggagttg ggagaccacc     420
   atcgccagcc ccagccgcat catctcccgc aaccgccgcc gccgccgcag ccacctgcaa     480
   ctttgcaggc gagagagcat cccgtctacc cgcctgagct gtccctcctg gacagcaccg     540
   acccacgcgc ctggctggct cccactttgc agggcatctg cacggcacgc gccgcccagt     600
   atttgctaca ttccccggag ctgggtgcct cagaggccgc tgcgccccgg gacgaggtgg     660
   acggccgggg ggagctggta aggaggagca gcggcggtgc cagcagcagc aagagccccg     720
   ggccggtgaa agtgcgggaa cagctgtgca agctgaaagg cggggtggtg gtagacgagc     780
   tgggctgcag ccgccaacgg gccccttcca gcaaacaggt gaatggggtg cagaagcaga     840
   gacggctagc agccaacgcc agggagcggc gcaggatgca tgggctgaac cacgccttcg     900
   accagctgcg caatgttatc ccgtcgttca acaacgacaa gaagctgtcc aaatatgaga     960
   ccctgcagat ggcccaaatc tacatcaacg ccttgtccga gctgctacaa acgcccagcg    1020
   gaggggaaca gccaccgccg cctccagcct cctgcaaaag cgaccaccac caccttcgca    1080
   ccgcggcctc ctatgaaggg ggcgcgggca acgcgaccgc agctggggct cagcaggctt    1140
   ccggagggag ccagcggccg accccgcccg ggagttgccg gactcgcttc tcagccccag    1200
   cttctgcggg agggtactcg gtgcagctgg acgctctgca cttctcgact ttcgaggaca    1260
   gcgccctgac agcgatgatg gcgcaaaaga atttgtctcc ttctctcccc gggagcatct    1320
   tgcagccagt gcaggaggaa aacagcaaaa cttcgcctcg gtcccacaga agcgacgggg    1380
   aattttcccc ccattcccat tacagtgact cggatgaggc aagttaggaa ggtgacagaa    1440
   gcctgaaaac tgagacagaa acaaaactgc cctttcccag tgcgcgggaa gccccgnggt    1500
   taangatccc cgcacccttt aatttnggct ctgcgatggt cgttgtttag caacgacttg    1560
   gctncagatg gt                                                         1572